(19) Europäisches Patentamt
European Patent Office
Office européen des brevets

(11) EP 1 427 441 B1

(12) **FASCICULE DE BREVET EUROPEEN**

(45) Date de publication et mention de la délivrance du brevet:
**01.12.2010 Bulletin 2010/48**

(21) Numéro de dépôt: **02772502.7**

(22) Date de dépôt: **09.08.2002**

(51) Int Cl.:
***A61K 39/21*** *(2006.01)*

(86) Numéro de dépôt international:
**PCT/FR2002/002853**

(87) Numéro de publication internationale:
**WO 2003/013593 (20.02.2003 Gazette 2003/08)**

(54) **SUPERIMMUNOGENE COMPOSITE A USAGE VACCINAL BIFONCTIONNEL Tat-gp160 POUR LE TRAITEMENT du SIDA.**

VERBUNDSUPERIMMUNOGEN ZUR VERWENDUNG ALS BIFUNKTIONELLER IMPFSTOFF (Tat-gp160) ZUR BEHANDLUNG VON AIDS

COMPOSITE SUPERIMMUNOGEN FOR BI-FUNCTIONAL VACCINE Tat-gp160 FOR USE IN THE TREATMENT OF AIDS

(84) Etats contractants désignés:
**AT BE BG CH CY CZ DE DK EE ES FI FR GB GR IE IT LI LU MC NL PT SE SK TR**

(30) Priorité: **10.08.2001 FR 0110751**

(43) Date de publication de la demande:
**16.06.2004 Bulletin 2004/25**

(73) Titulaire: **NEOVACS**
**75016 Paris (FR)**

(72) Inventeurs:
- **ZAGURY, Daniel**
  **F-75007 Paris (FR)**
- **BIZZINI, Bernard**
  **F-81000 Albi (FR)**
- **ZAGURY, Jean François**
  **F-75003 Paris (FR)**
- **LE BUANEC, Hélène**
  **F-75005 Paris (FR)**

(74) Mandataire: **Catherine, Alain**
**Cabinet Harlé & Phélip**
**7 rue de Madrid**
**75008 Paris (FR)**

(56) Documents cités:
**WO-A-00/03732** **WO-A-00/78334**
**WO-A-02/11759** **WO-A1-00/23955**
**WO-A1-00/23955** **WO-A1-96/26277**
**WO-A1-96/27389** **WO-A2-01/54719**
**US-A- 6 093 405**

- **ZAGURY D ET AL: "Toward a new generation of vaccines: The anti-cytokine therapeutic vaccines" PROCEEDINGS OF THE NATIONAL ACADEMY OF SCIENCES OF USA, NATIONAL ACADEMY OF SCIENCE. WASHINGTON, US, vol. 98, no. 14, 3 juillet 2001 (2001-07-03), pages 8024-8029, XP002186083 ISSN: 0027-8424**
- **GRINGERI A ET AL: "SAFETY AND IMMUNOGENICITY OF HIV-1 TAT TOXOID IN IMMUNOCOMPROMISED HIV-1-INFECTED PATIENTS" JOURNAL OF HUMAN VIROLOGY, LIPPINCOTT WILLIAMS & WILKINS, US, vol. 1, no. 4, 1 May 1998 (1998-05-01), pages 293-298, XP001133876 ISSN: 1090-9508**
- **CARELLI C ET AL: "Immunogenicity of combined anti-HIV and anti-suppressive vaccine preparations" BIOMEDICINE AND PHARMACOTHERAPY, ELSEVIER, PARIS, FR LNKD- DOI:10.1016/0753-3322(92)90022-Y, vol. 46, no. 4, 1 January 1992 (1992-01-01), pages 149-153, XP023745371 ISSN: 0753-3322 [retrieved on 1992-01-01]**
- **GRINGERI ET AL: "Safety and immunogenicity of HIV-1 Tat Toxoid in Immunocompromised HIV-1-Infected Patients" JOURNAL OF HUMAN VIROLOGY, vol. 1, no. 4, 1998, pages 293-298,**
- **CARELLI ET AL: "Immunogenicity of combined anti-HIV and anti-suppressive vaccine preparation" BIOMED & PHARMACOTHER, vol. 46, 1992, pages 149-153,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

Remarques:

Le dossier contient des informations techniques présentées postérieurement au dépôt de la demande et ne figurant pas dans le présent fascicule.

**Description**

## DOMAINE DE L'INVENTION

La présente invention se rapporte à la prévention et au traitement de pathologies provoquées par l'expression tissulaire locale d'une structure antigénique pathogène, expression associée à un dérèglement stromal d'ordre immunitaire ou vasculaire entraînant une immunotoxicité ou une néoanglogénèse, ces pathologies englobant certaines infections par des virus, certains cancers et les allergies.

Elle est relative à la mise au point de nouveaux moyens thérapeutiques préventifs ou curatifs, désignés composés superimmunogènes composites, induisant une réaction immunitaire à la fois à l'encontre de la structure antigénique pathogène et à l'encontre de la protéine ou des protéine(s) causales du dérèglement tissulaire stromal associé.

## ART ANTERIEUR

A la suite des premiers travaux expérimentaux de Louis Pasteur, on a recherché, durant le vingtième siècle, à comprendre les mécanismes de l'immunité afin de préparer des vaccins de spécificité et d'efficacité toujours plus grands. Les premiers vaccins utilisés à grande échelle ont consisté en des microbes vivants atténués ou en des préparations immunogènes associées à des impuretés protéiques ou membranaires de composition et de structure mal caractérisées agissant comme adjuvants de l'immunité.

Puis, on a mis au point des vaccins préparés à partir d'antigènes purifiés, tels que des sous-unités protéiques ou des protéines toxoïdes, associés à des adjuvants de l'immunité mieux définis, plus efficaces et dépourvus de toxicité, essentiellement destinés à combattre et contrôler des maladies infectieuses par ciblage de la réaction immunitaire à l'encontre de l'agent infectieux causal.

Pendant les deux dernières décennies, le succès des vaccinations à grande échelle, y compris par les techniques du génie génétique, ainsi qu'une meilleure compréhension des mécanismes de la réaction immunitaire, a permis aux chercheurs d'étendre le recours à la vaccination en vue de traiter des maladies chroniques associées à des structures biologiques (les microbes, cellules ou particules de l'environnent inertes ou vivantes) porteuses d'antigènes étrangers ou encore d'antigènes anormalement exprimés, comme dans les pathologies du SIDA, des cancers, de l'allergie et des maladies auto-immunes.

Pour prévenir ou traiter les pathologies ci-dessus au moyen de la vaccination, on a recherché, de manière systématique, à induire une réaction immunitaire ciblée sur la structure pathogène, par exempte des protéines virales exprimées sélectivement par les cellules infectées par un virus, les protéines exprimées sélectivement par les cellules cancéreuses ou encore les protéines allergènes, qui sont les agents causals primaires de ces maladies.

A titre illustratif, les vaccins candidats actuellement préparés en vue de combattre l'infection par les virus du SIDA, en particulier par le virus HIV1, visent à provoquer l'induction d'une réaction immunitaire exclusivement ciblée contre certaines protéines ou peptides viraux.

De même, les vaccins anti-cancer faisant l'objet des études cliniques les plus avancées visent à induire une réaction immunitaire ciblant exclusivement la destruction des cellules exprimant des antigènes associés au cancer, tels des protéines virales dans le cas de cancers provoqués par certains papillomavirus, ou la destruction des cellules infectées par un virus, tel le HIV1 dans la maladie du SIDA.

Selon une même stratégie vaccinale, les vaccins anti-allergiques actuels visent exclusivement à induire une réaction immunitaire ciblée sur l'allergène causal primaire

Les cancers sont des proliférations de cellules qui peuvent ensuite essaimer dans l'organisme pour former des métastases. Il est connu que le système immunitaire d'un individu normal élimine régulièrement les cellules cancéreuses naissantes, et que la formation d'un cancer est associée (1) à l'échappement du système de surveillance immunitaire locale puis, à une période avancée du cancer, à une immunosuppression systémique et (2) à une prolifération des cellules endothéliales vasculaires assurant l'apport nutritif des cellules tumorales, cette prolifération des cellules endothéliales étant désignée néoangiogénèse.

Le phénomène d'échappement aux défenses immunitaires cellulaires de l'hôte par induction de leur paralysie *in situ* est une stratégie utilisée par de nombreux cancers et est nécessaire à leur survie. Initialement, l'immunosuppression reste localisée au niveau de la tumeur, puisque l'individu est encore capable de se défendre contre les autres agressions telles que des infections.

Cependant, à un stade plus tardif, cette immunosuppression peut s'étendre, se généraliser, ainsi que l'atteste la dissémination de métastases et la grande vulnérabilité du cancéreux face aux infections. Cette immunosuppression met en jeu des facteurs paralysants qui sont produits par les cellules cancéreuses ou par les cellules de leur environnement. La paralysie locale des cellules du système immunitaire, ou immunosuppression, représente donc une arme majeure des cellules cancéreuses qui leur permet d'échapper au système immunitaire de l'hôte. Cette même stratégie immunosuppressive est également utilisée par l'agresseur viral, dans certaines maladies infectieuses, tel le SIDA. Ainsi,

des protéines relâchées par les cellules infectées par le HIV1 agissent comme de véritables toxines sur les cellules immunitaires environnantes, les dérèglent et bloquent *in situ,* c'est-à-dire de manière paracrine, les cellules du système immunitaire, protégeant les cellules infectées, la réplication des virus et leur dissémination.

Des travaux antérieurs de la demanderesse, relatés dans la demande Internationale publiée sous le n°WO 00/03732, ont montré que, dans le cas de la leucémie ATL, du cancer du col utérin, et du sarcome de Kaposi, respectivement trois protéines étaient impliquées dans une immunosuppression locale au niveau de tumeurs ou de cellules infectées par le VIH1:

la protéine Tax du virus HTLV 1,
la protéine E7 du papillomavirus et
la protéine Tat du virus VIH-1.

La demanderesse avait aussi décrit que certaines de ces protéines immunosuppressives, telles la protéine Tat du HIV1 et la protéine E7 de HPV (souches 16 et 18) ont également des effets activateurs sur les cellules endothéliales vasculaires.

Elle avait en conséquence proposé la mise au point de vaccins anti-cancer ou anti-viraux comprenant un composé immunogène dérivé détoxiqué d'une protéine provenant de cellules cancéreuses, de cellules infectées par un virus ou de cellules immunitaires stromales, initialement immunosuppressive et/ou angiogénique à action locale, comme par exemple une protéine dérivée de la protéine Tat du virus HIV1, la protéine Tax d'un virus HTLV1, la protéine E7 d'un papillomavirus ou encore une lectine mannane-dépendante, sous une forme inactivée.

Selon un autre aspect d'une stratégie thérapeutique potentielle pour lutter contre le SIDA, le cancer et les allergies, basée sur un principe proche des vacins proposés dans la demande PCT n° WO 00/03732 ci-dessus, ZAGURY D et al. (2001, Proc. Natl. Acad. Sci. USA, 98(14) : 8024-8029), dans une étude bibliographique, suggèrent d'induire une immunité anti-cytokine chez les patients afin de contrecarrer la production anormale dans ces pathologies de certaines cytokine, notamment des interleukines, lymphokines, monokines, interférons qui agissent physiologiquement dans les tissus, localement comme facteur de prolifération, de différenciation ou de mort programmée cellulaire.

Ces auteurs précisent que les stratégies de thérapeutique vaccinale ont été, jusqu'à aujourd'hui, exclusivement ciblées sur l'agresseur antigénique, que ce soit un microorganisme, une cellule ou un allergène, mais n'ont jamais recherché à combattre la dérégulation des cytokines induites sous l'effet de l'agresseur. Ces auteurs proposent une vaccination anti-cytokine en tant qu'auxiliaire d'une vaccination conventionnelle dont le but serait de neutraliser ou bloquer les effets immunotoxiques du stroma, et de permettre le déroulement normal de la réaction immunitaire adaptée contre l'agresseur antigénique. Toutefois, ZAGURY et al. (2001) n'apportent aucune démonstration expérimentale concrète de nature à prouver le bénéfice que l'induction d'une telle réponse immunitaire pourrait apporter pour les patients infectés par un virus, les patients cancéreux ou encore sujets à des réactions allergiques graves, par exemple systémiques.

Il existe un besoin dans l'état de la technique pour des moyens permettant une thérapeutique vaccinale améliorée, à la fois plus sûre et plus efficace que les thérapeutiques vaccinales actuelles, afin de prévenir ou traiter certains cancers, les infections virales, notamment par les virus HTLV-1 ou HIV-1, ou les allergies graves.

## SOMMAIRE DE L'INVENTION

Un nouveau moyen de thérapeutique vaccinale systémique ou mucosale contre le SIDA sont fournis par l'invention sous la forme de composés superimmunogènes composites à usage vaccinal bifonctionnel comprenant la protéine gp160 et Tat du virus HIV1 comme défini dans les revendications.

L'utilisation d'un superimmunogène composite bifonctionnel comprenant deux polypeptides immunogènes distincts physiquement liés l'un à l'autre est décrite, les deux polypeptides consistant respectivement en :

(a) un premier polypeptide immunogène induisant une réaction immunitaire cellulaire, ou une réaction immunitaire cellulaire et humorale, à l'encontre d'une structure antigénique pathogène cellulaire, microbienne ou particulaire inerte ou vivante ;

(b) un second polypeptide immunogène induisant la production d'anticorps neutralisants ou bloquants à l'encontre d'une protéine circulante locale du stroma choisie parmi un facteur cytokinique ou un facteur de régulation cellulaire à propriétés immunotoxiques ou angiogéniques, ce facteur pouvant être soit produit par les cellules cancéreuses, les cellules infectées par un virus ou les cellules stromales, y compris les lymphocytes T et les cellules présentant l'antigène (APC), soit induit par des structures particulaires pathogènes, notamment allergéniques,

pour l'obtention d'un médicament à action anti-cancéreuse, anti-virale ou anti-allergique induisant une immunité mucosale ou systémique simultanément à l'encontre de la structure antigénique pathogène et de la protéine circulante locale du stroma.

Selon un premier aspect, le polypeptide (a) induit une réaction immunitaire cellulaire, et éventuellement aussi une réaction immunitaire humorale, à l'encontre d'un antigène exprimé spécifiquement par les cellules cancéreuses, les cellules infectées par un virus ou un antigène constitutif des structures particulaires, incluant les structures particulaires vivantes, comme le pollen, les acariens et certains parasites comme les *Leishmania major,* et les structures particulaires inertes, comme la poussière, ou les poils de chat.

Le premier polypeptide (a) immunogène est choisi parmi (i) une protéine immunogène sélectivement exprimée par les cellules cancéreuses, sélectivement exprimée par les cellules infectées par un virus ou constitutive d'une structure pathogène allergène, le cas échéant détoxiquée, et (ii) une protéine dérivée de la protéine (i).

Selon un second aspect, le polypeptide (b) induit une réaction humorale à l'encontre de la protéine circulante libérée localement dans le stroma.tissulaire de manière anormale

Selon ce second aspect, le polypeptide (b) immunogène est choisi parmi (i) la protéine circulante locale du stroma, le cas échéant détoxiquée, et (ii) une protéine dérivée de la protéine (i).

On précise que certaines protéines à propriétés immunotoxiques ou angiogéniques, qui sont constitutives de structures pathogènes cellulaires et qui sont libérées dans le stroma, peuvent être utilisées comme polypeptide (a) ou polypeptide (b) d'un superimmunogène composite. C'est le cas pour la protéine Tat du HIV, de la protéine E7 de l'HPV ou encore du facteur de régulation cellulaire p53.

On utilise de préférence les composés superimmunogènes suivants :

*a) Vaccination anti-virale*

- le superimmunogène composite (a) gp160 - (b) Tat toxoïde du HIV1;
- le superimmunogène composite (b) peptide Tat [1-15 ;46-60] - (a) gp160 du HIV1;
- le superimmunogène composite (a) Tat toxoïde du HIV1- (b) IFN$\alpha$ ;
- le superimmunogène composite (a) Tat toxoïde - (b) peptide Tat [1-15 ;46-60] du HIV1

*b) Vaccination anti-cancer*

- le superimmunogène composite (a) L1 - (b) E7 du HPV;
- le superimmunogène composite (a) E7 du HPV - (b) VEGF ;

*c) Vaccination anti-allergène :*

- le superimmunogène composite (a) Betv1a - (b) IL4 hétérologue

Les polypeptides (a) et (b) sont liés physiquement l'un à l'autre en ce qu'ils sont, dans tous les cas, présentés conjointement aux cellules du système immunitaire sur un support unique. Les polypeptides (a) et (b) peuvent être liés de manière covalente sur la même structure moléculaire.

Les polypeptides (a) et (b) peuvent aussi être inclus tous les deux au sein d'une même structure physique par exemple sur des monoparticules ayant un diamètre compris entre 10 et 500 nanomètres de préférence 10 et 1000 nanomètres, et de manière tout à fait préférée entre 10 et 100 nanomètres, par exemple des nanopaticules d'IMS, comme décrit par exemple par Aucouturier et al. (2001), de chitosan , comme décrit par exemple par Sjaugrud et al. (1999), de liposomes, ou de particules biodégradables comme le polylactide acide (PLA), le poly-$\varepsilon$-caprolactone (PCL) ou le poly(lactide-coglycolide) (PLG) décrit par Baras et al. (1999).

Selon un premier aspect, l'utilisation est caractérisée en ce que les polypeptides (a) et (b) sont liés directement entre eux de manière covalente.

Selon un second aspect, l'utilisation est caractérisée en ce que les polypeptides (a) et (b) sont séparés l'un de l'autre, au sein du superimmunogène peptidique, par une chaîne espaceur. La chaîne espaceur peut notamment consister en un peptide espaceur linéaire, une peptide espaceur ramifié, ou encore un composé espaceur bifonctionnel que le SMCC ou le SIAB.

Selon un troisième aspect, les polypeptides (a) et (b) sont immobilisés sur des nanoparticules ou enchâssés dans des microparticules ou dans des nanoparticules. De préférence, le polypeptide (a) et le polypeptide (b) sont immobilisés tous les deux sur la même nanoparticule, ou enchâssées dans la même nanoparticule.

Egalement décrit est un conjugué peptidique immunogène comprenant deux polypeptides distincts liés physiquement, l'un à l'autre, les deux polypeptides consistant respectivement en les polypeptides (a) et (b) définis précédemment.

La description concerne aussi un acide nucléique codant un composé superimmunogène composite tel que défini ci-dessus, ainsi qu'une cassette d'expression et un vecteur recombinant comprenant un tel acide nucléique, ainsi que l'utilisation de cet acide nucléique, de cette cassette d'expression ou de ce vecteur recombinant pour l'obtention

d'un médicament à action anti-cancéreuse, anti-virale ou anti-allergique.

Elle a également trait à une composition immunogène comprenant une quantité immunologiquement efficace d'un composé immunogène tel que défini ci-dessus, en association avec un ou plusieurs excipients, dont des adjuvants d'immunité, physiologiquement compatibles.

Selon les objectifs poursuivis, on utilise des adjuvants systémiques ou des adjuvants mucosals. Par exemple, on utilise de préférence un adjuvant mucosal pour prévenir les cancers des tissus épithéliaux et on utilise de préférence des adjuvants systémiques pour prévenir ou traiter les infections par des virus, tels que par HIV1 et HTLV1, ou encore pour prévenir ou traiter les allergies.

Parmi les adjuvants systémiques, on utilise de préférence les adjuvants de type IFA (Adjuvant Incomplet de Freund), le phosphate de calcium ou l'hydroxyde d'alumine.

Parmi les adjuvants mucosals, on utilise de préférence des adjuvants comme la choleratoxine B (CTB) ou un mutant de la toxine labile de *Escherichia coli* LT (LT$\mu$).

Elle concerne aussi un vaccin, mucosal ou systémique, caractérisé en ce qu'il comprend, à titre de principe actif, un composé immunogène tel que défini ci-dessus, en association avec un ou plusieurs excipients, dont des adjuvants d'immunité, physiologiquement compatibles.

La description a aussi pour objet une composition immunogène et un vaccin à visée mucosale ou systémique, caractérisés en ce qu'ils comprennent une quantité thérapeutiquement efficace d'un acide nucléique, d'une cassette d'expression ou d'un vecteur recombinant tels que définis ci-dessus.

## DESCRIPTION DE LA FIGURE UNIQUE

La **Figure 1** est un schéma général de la stratégie vaccinale mise au point grâce aux composés superimmunogènes composites.

La flèche représentée sur le côté gauche de la Figure 1 désigne la nature des structures ciblées par les préparations vaccinales conventionnelles anti-cancer, anti-virales et anti-allergiques, c'est à dire les structures antigéniques pathogènes représentant les agents causals primaires du développement de la pathologie, comme les cellules cancéreuses, les cellules infectées par le HIV ou les antigènes allergènes des pollens.

La double flèche représentée sur le côté droit de la figure 1 désigne la nature des structures ciblées par les préparations vaccinales selon l'invention, qui sont de deux types :

a) les structures antigéniques pathogènes détaillées ci-dessus ; et

b) les facteurs protéiques circulant dans le micro-environnement stromal responsables du dérèglement stromal tissulaire d'ordre immunitaire et vasculaire observé dans certains cancers, infections virales et allergies graves.

## DESCRIPTION DETAILLEE DE L'INVENTION

Il a été découvert par le demandeur que l'immunosuppression et l'angiogénèse du micro-environnement des cellules infectées par certains virus tels que le VIH-1 et du micro-environnement des cellules cancéreuses apportent une explication rationnelle à l'absence d'efficacité des stratégies vaccinales de l'état de la technique, qui ciblent exclusivement la cellule cancéreuse et non le dérèglement de son micro-environnement.

Les travaux de la demanderesse ont montré que des facteurs solubles sécrétés par les cellules infectées par le VIH-1, en particulier la protéine Tat ou par les cellules immunitaires de patients infectés par le VIH, en particulier l'IFN-$\alpha$ et le TGF-$\beta$, ou produit par des cellules cancéreuses, telles la protéine E7 de l'HPV dans le cancer du col utérin ou la protéine Tax du HTLV1 dans les leucémies ATL , avaient des propriétés immunosuppressives susceptibles d'inhiber les réactions immunitaires cellulaires au sein des tissus infectés ou des tumeurs et de ce fait expliquer une absence d'efficacité des vaccins conventionnels, qui utilisent exclusivement des immunogènes portés par des structures antigéniques pathogènes, comme les cellules cancéreuses, les cellules infectées par le VIH.

L'étude bibliographique a permis de conforter les observations de la demanderesse, en confirmant la présence de facteurs immunosuppressifs relâchés dans le milieu extracellulaire de tumeurs malignes.

Certains de ces facteurs, non encore identifiés, ont été produits par:

- des cellules de cancer colorectal (Ebert EC, Roberts Al, O'Connell SM, Robertson FM, Nagase H. Characterization of an immunosuppressive factor dérived from colon cancer cells. J. Immun ol. (1987) 138.2161-8 ou Remacle-Bonn et MM, Pommier FJ, Kaplanski S, Rance RJ, Depieds RC. Inhibition of normal allogenic lymphocyte mitogenesis by a soluble inhibitor extracted from human colonic carcinoma J. Immunol. (1976) 117:1145-51,
- des cellules de glioblastome (29-Fontana A, Hengartner H, de Tribolet N, Weber E. Glioblastoma cells release interleukin 1 and factors inhibiting interleukin 2-mediated effects. J. Immunol. (1984) 132:1837-44),
- des melanomas (30.Hersey P. Bindon, Czemiecki M, spurling A, Wass J, McCarthy WH. Inhibition of interleukin 2

production by factors released from tumor cells J. Immunol. (1983) 131:2837-42), ou

- des ascites malignes (Tamura K, Shibata Y, Matsuda Y, Ishida N. Isolation and characterization of an immunosuppressive acidid protein from ascitic fluids of cancer patients. Cancer Res. (1981) 41:3244-52, Oh SK, Moolten FL. Non specific immunosuppressive factors in malignant ascites : further characterization and possible relationship to erythrocyte receptors of human peripheral Moolten FL. Non specific immunosuppressive factors in malignant ascites: further characterisation and possible relationship to erythrocyte receptors of human peripheral T cells. J. Immunol (1981) 127:2300-7).

D'autres facteurs de regulation transcriptionnelle, comme rapporté plus haut, sont d'origine cellulaire telle la protéine p53, accumulée dans certaines tumeurs malignes, en particulier colorectale (Remvikos Y. Tominaga O, Hammel P, Laurent-Puig P, Salmon RJ, Dutrillaux B, Thomas G. Increased p53 protein content of colorectal tumours correlates with poor survival. Br J Cancer 1992 66 :758-64, Gan H, Ouyang Q, Wang Y. Expression of p53 protein in colorectal cancer and its relationship to cell proliferative activity and prognosis. Chung Hua Chung Liu Tsa Chih (1996). La protéine p53 relâchée par transport actif par des voies de sécrétion n'utilisant pas le signal peptide ou par diffusion passive est présente dans le milieu extracellulaire, et elle a été isolée par chromatographie sur fibre de verre à partir de sérum de cancéreux (Zusman I, Sandler B, Gurevich P, zusman R, Smimoff P, Tendler Y, Bass D, Shani A, Idelevich E, Pfefferman R, Davidovich B, Huszar M, Glick J. Comparative study of the role of serum levels of p53 antigen and its tumor cell concentration in colon cancer detection. Hum Antibodies Hybridomas. (1996) : 123-8, Sandler B, Smimoff P, Tendelr Y, Zinder O, Zusman R, Zusman I. Specificity of polyclonal anti-p53 IgG for isolation of the soluble p53 antigen from human serum. Int J. Mol. Med. 1998 1:767-70).

Des cytokines, telles le TGFβ notoirement immunosuppressif, le VEGF facteur de croissance angiogénique, l'IL-6 proinflammatoire ou l'IL10 également immunosuppressive, sont anormalement secrétées et relâchées dans le milieu extracellulaire de certaines cellules cancéreuses. La demanderesse a elle-même montré que les cellules de lignée cancéreuses SIHA, tout comme les cellules DU145 du cancer de la prostate et les cellules MT2 de lignées leucémiques produisent anormalement et relâchent dans le milieu extracellulaire des cytokines telles le VEGF et/ou l'IL6 tandis que les cellules RAJI de lignées leucémiques secrètent dans le milieu extracellulaie de l'IL10.

Il est désormais montré que, pour la prévention ou le traitement de certains cancers, de certaines infections virales et des allergies, en particulier les allergies graves comportant un risque de choc anaphylactique, il était essentiel d'induire une réaction immunitaire simultanément :

a) à l'encontre d'une structure antigénique pathogène, cellulaire, microbienne ou particulaire, constituant l'agent causal primaire de la maladie; et

b) à l'encontre d'une protéine circulante locale du stroma responsable du dérèglement stromal tissulaire d'ordre immunitaire ou vasculaire associé à la maladie.

Il est montré que la réaction immunitaire définie ci-dessus peut être induite selon l'invention, à haut niveau de stimulation des cellules du système immunitaire, grâce à une famille de composés désignés superimmunogènes composites, qui sont bifonctionnels, comprenant, sous une forme physiquement liée entre eux, un premier polypeptide induisant une réaction immunitaire contre la structure antigénique pathogène et un second polypeptide induisant une réaction immunitaire à l'encontre de la protéine circulante locale du stroma.

Par « stroma », on entend, pour un tissu donné, les espaces intercellulaires constituant le micro-environnement des cellules spécialisées d'un tissu. Dans le tissu hépatique, le stroma environnant les cordons des cellules hépatiques spécialisées contient un milieu nutritif, aussi appelé lymphe, qui héberge à la fois des cellules immunitaires (lymphocytes T et B, cellules présentant l'antigène ou APC), des polynucléaires, des mastocytes, des fibroblastes et des vaisseaux capillaires tapissés de cellules endothéliales. Dans les tissus épithéliaux muqueux ou cutanés, le stroma sous-épithélial, appelé derme, est aussi formé d'espaces intercellulaires qui se rattachent au tissu conjonctif qui en constitue la matrice et dans lesquels circulent aussi les cellules du système immunitaire et les vaisseaux capillaires précités.

Pour prévenir ou traiter certains cancers, les superimmunogènes composite induisent simultanément :

a) une réaction immunitaire de type cellulaire, par l'activation de cellules T auxiliaires (Cellules « T helper » ou « Th ») et/ou production de cellules T cytotoxiques (CTL) spécifiques de la structure antigénique pathogène exprimée par les cellules cancéreuses, par exemple les antigènes de tumeurs TAA ou TSA, ou encore la production de cellules tueuses dites « Natural Killer » ou « NK » permettant de tuer sélectivement lesdites cellules cancéreuses; et

b) une réaction immunitaire humorale, par l'induction de la production d'anticorps neutralisants ou bloquants spécifiques d'une protéine circulante locale du stroma anormalement produite, principalement une protéine à propriétés immunotoxiques ou à propriétés angiogéniques.

Pour prévenir ou traiter certaines infections virales, le cas échéant associées au développement de cancers,

les superimmunogènes composites induisent simultanément :

a) une réaction immunitaire de type cellulaire, par l'activation de cellules T auxiliaires et/ou par l'induction de la production de cellules T cytotoxiques (CTL) spécifiques de la structure antigénique pathogène virale exprimée par les cellules infectées par un virus, comme certaines protéines de HIV1 ou du papillomavirus, ou encore la production de cellules tueuses dites « Natural Killer » ou « NK » permettant de tuer sélectivement lesdites cellules infectées par le virus; et
b) une réaction immunitaire humorale, par l'induction de la production d'anticorps neutralisants ou bloquants spécifiques d'une protéine circulante locale du stroma anormalement produite, y compris une protéine circulante virale ou une cytokine, principalement une protéine à propriétés immunosuppressives ou apotogènes (immunotoxiques) ou à propriétés angiogéniques.

De plus, le demandeur a rapproché les données concernant l'observation d'une immunotoxicité dans le cas d'infections par le virus VIH1 ou le virus HTLV1 avec les observations de déviations de réactions immunitaires induite par la production anormale du facteur cytokinique IL4 lors d'immunisations dirigées contre des structures particulaires du pollen, d'acariens ou parasitaires, tels le Leishmania major qui ont été rapportées par Sadick et al. (1990, J. Exp. Med., 171 : 115-127).
Pour prévenir ou traiter les allergies, surtout les allergies graves, les superimmunogènes composites induisent simultanément :

a) une réaction immunitaire cellulaire par l'activation de cellules T auxiliaires et une réaction immunitaire humorale, par l'induction d'anticorps spécifiques de la structure antigénique pathogène allergène constitutive de la particule allergénique.
b) une réaction immunitaire humorale, par l'induction de la production d'anticorps neutralisants ou bloquants spécifiques d'une protéine circulante locale du stroma anormalement produite, principalement le facteur cytokinique IL4 synthétisé notamment par les lymphocytes T de type Th2. La surproduction d'IL4, qui est un co-stimulus de la production d'IgE par les lymphocytes B, est également immunotoxique en ce qu'elle induit des réponses immunitaires pathogènes, de type inflammatoire allergique.

La description a pour objet l'utilisation d'un superimmunogène composite comprenant deux polypeptides immunogènes distincts physiquement liés l'un à l'autre, les deux polypeptides consistant respectivement en :

(a) un premier polypeptide immunogène induisant une réaction immunitaire cellulaire, ou une réaction immunitaire cellulaire et humorale, à l'encontre d'une structure antigénique pathogène cellulaire, microbienne ou particulaire inerte ou vivante ;
(b) un second polypeptide immunogène induisant la production d'anticorps neutralisants ou bloquants à l'encontre d'une protéine circulante locale du stroma choisie parmi un facteur cytokinique ou un facteur de régulation cellulaire à propriétés immunotoxiques ou angiogéniques, ce facteur pouvant être soit produit par les cellules cancéreuses, les cellules infectées par un virus ou les cellules stromales, y compris les lymphocytes T et les cellules présentant l'antigène (APC), soit induit par les structures particulaires pathogènes, notamment allergéniques.

Le superimmunogène composite utilisé est dit « bifonctionnel » car les deux polypeptides (a) et (b), qui sont les deux parties essentielles qui le constituent, permettent l'induction simultanée d'une réaction immunitaire dirigée contre deux cibles distinctes, respectivement la structure antigénique pathogène et la protéine circulante locale du stroma. Toutefois, un superimmunogène composite bifonctionnel peut comprendre dans sa structure une pluralité de copies respectivement d'un polypeptide (a) et/ou d'un polypeptide (b).
Le polypeptide (a) et le polypeptide (b) constitutifs d'un composé superimmunogène composite sont dits « physiquement liés » l'un à l'autre car ils sont dans tous les cas inclus tous les deux dans une même structure physique, molécule ou particule (microparticule ou nanoparticule), au sein de laquelle ils sont peu distants l'un de l'autre. Du fait qu'ils sont physiquement liés l'un à l'autre dans le superimmunogène composite, le polypeptide (a) et le polypeptide (b) sont présentés conjointement aux mêmes cellules immunocompétentes, aussi bien les macrophages que les lymphocytes T ou B.
Par réaction immunitaire « cellulaire », on entend une activation :

- soit des lymphocytes T auxiliaires (Th) possédant un récepteur reconnaissant spécifiquement le polypeptide (a) ou le polypeptide (b) en association avec un antigène de Classe II du Complexe majeur d'histocompatibilité (CMH);
- soit des lymphocytes T cytotoxiques (CTL) possédant un récepteur à l'antigène reconnaissant spécifiquement le polypeptide (a) en association avec un antigène de Classe I du MHC , et éventuellement de cellules NK.

La réaction cellulaire pourra se mesurer *in vitro* chez la souris par la production de chimiokines (le MIP1α et le MIP1β) par les splénocytes et par la prolifération de ces splénocytes activés en présence de ces immunogènes, ou encore par l'augmentation de la production d'IFNγ par ces cellules, illustrant l'activité T cytotoxique.

Par réaction immunitaire « humorale », on entend la production d'anticorps spécifiquement à l'encontre du polypeptide (a) ou du polypeptide (b) constitutifs du superimmunogène composite.

Par « structure antigénique pathogène cellulaire, microbienne ou particulaire », on entend :

- soit une cellule cancéreuse exprimant sélectivement un antigène, qui peut être désigné « antigène de tumeurs », et qui peut être tuée par une réaction immunitaire cellulaire, et préférentiellement par des cellules cytotoxiques (CTL) reconnaissant spécifiquement l'antigène porté sélectivement par cette cellule cancéreuse, ou qui peut être tuée par des cellules NK activées ;
- soit une cellule infectée par un virus exprimant sélectivement un antigène viral, et qui peut être tuée par une réaction immunitaire cellulaire, et préférentiellement par des cellules cytotoxiques (CTL) reconnaissant spécifiquement l'antigène exprimé sélectivement par cette cellule infectée, ou qui peut être tuée par des cellules NK activées ;
- soit une particule allergénique comprenant un antigène allergène qui peut être bloqué ou inactivé par un anticorps dirigé spécifiquement contre cet antigène allergène, ledit anticorps étant produit durant une réaction immunitaire humorale. Les particules allergéniques englobent les particules allergéniques vivantes, comme les pollens ou les parasites comme les acariens ou les *Leishmania major,* et les particules allergéniques inertes, comme les particules de poussière ou les poils de chat..

Les protéines «à propriétés immunotoxiques » anormalement libérées dans le stroma englobent :

- les protéines à propriétés immunosuppressives, comme la protéine Tat de HIV1, la protéine E7 du papillomavirus, l'interféron alpha (IFNα), le TGFβ ou l'IL10, la protéine p53 sous sa forme circulante ou encore la cytokine apoptogène Fas ligand (FasL) ;
- les protéines déviant la réaction immunitaire vers une action délétère, telle que le facteur cytokinique IL4 qui stimule la production d'anticorps d'isotype IgE, responsables notamment de la libération d'histamine par les mastocytes, pouvant entraîner une action délétère inflammatoire forte, voire mortelle pour l'organisme, sous la forme d'un choc anaphylactique.

Les protéines « à propriétés angiogéniques » sont les protéines induisant la multiplication des cellules endothéliales, comme le facteur cytokinique VEGF. Dans le cas de tumeurs de cellules cancéreuses, la libération des protéines à propriétés angiogéniques, localement, dans le stroma environnant la tumeur, induit une prolifération des cellules endothéliales vasculaires lors d'un événement appelé « néoangiogénène » qui va permettre une vascularisation de la tumeur nécessaire à sa survie, notamment par l'apport des éléments nutritifs requis pour la viabilité et la multiplication des cellules cancéreuses constitutives de la tumeur.

Par immunité « mucosale », on entend l'induction d'une réaction immunitaire humorale aboutissant à la production principalement d'anticorps d'isotype IgA produits par les lymphocytes B localisés au niveau des tissus épithéliaux, appelés anticorps IgA sécrétoires (s-IgA) et d'une réaction immunitaire cellulaire au sein des ganglions drainant les muqueuses (ganglions mésentériques pour les intestins, ganglions iliaques pour le vagin).

Par immunité « systémique », on entend l'induction d'une réaction humorale aboutissant à la production principalement d'anticorps d'isotype IgG, qui circulent de manière systémique, notamment par la voie sérique et d'une réaction immunitaire cellulaire au sein des ganglions périphériques ou de la rate..

Il est montré que, pour une diversité de composés superimmunogènes composites tels que définis ci-dessus, - le niveau de réponse immunitaire obtenu *in vivo* est bien supérieur à la réponse immunitaire observée après administration de chacun des polypeptides (a) et (b), non liés physiquement l'un à l'autre.

Sans vouloir être liés par une quelconque théorie, les inventeurs pensent que le polypeptide (a) apporte, au sein du superimmunogène composite, des épitopes T auxilliaires qui vont stimuler un effet « helper » et ainsi activer, ou augmenter l'activation de la réaction immunitaire humorale à l'encontre du polypeptide (b). Cette action auxiliaire du polypeptide (a) immunogène est particulièrement nécessaire pour induire la production d'anticorps dirigés contre le polypeptide (b), lorsque le polypeptide (b) est un facteur cytokinique autologue, comme par exemple l'IL4 humaine dans un superimmunogène composite destiné à l'administration chez l'homme.

Il est montré que la production d'anticorps de type IgG dirigés contre la protéine Tat native est deux fois plus importante lorsque le conjugué peptidique gp160-Tat inactivé (toxoïde) est administré *in vivo* qu'en réponse à l'administration de la protéine Tat inactivée (toxoïde) sous forme libre. De plus, les anticorps dont la production est induite par le superimmunogène composite gp160-Tat(toxoïde) permettent de neutraliser 100% de l'activité de la protéine Tat native, alors qu'une neutralisation maximale de 75% de l'activité de la protéine Tat native est observée avec les anticorps produits après administration de la protéine Tat (toxoïde) sous forme libre.

Egalement, les résultats des exemples montrent que les splénocytes d'animaux immunisés avec le superimmunogène composite gp160-Tat(toxoïde) produisent un niveau plus élevé de chimiokines MIP1α et MIP1β que les cellules des animaux immunisés par le Tat(toxoïde), ce qui montre qu'une stimulation optimale des cellules immunitaires est obtenue avec un superimmunogène composite conforme à l'invention.

Les splénocytes d'animaux immunisés avec le superimmunogène composite gp160-Tat (toxoïde) produisent aussi une quantité importante d'interféron-γ lorsqu'elles sont activées, *in vitro,* par la protéine Tat native.

Des résultats similaires ont été obtenus avec d'autres superimmunogènes composites conformes à l'invention, tels que le conjugué E7-SIAB-VEGF, ou encore le conjugué Betv1a-IL4.

Ainsi, l'utilisation d'un superimmunogène composite tel que défini ci-dessus peut, au moins dans certains cas, potentialiser l'effet thérapeutique qui serait observé par l'utilisation séparée de chacun des polypeptides (a) et (b) ou encore par l'utilisation d'une association des polypeptides (a) et (b) non physiquement liés l'un à l'autre, ceci à au moins à deux titres :

(i) l'induction d'une réponse immunitaire efficace, de type cellulaire ou humorale selon le cas, à l'encontre d'un antigène exprimé par les cellules tumorales ou d'un antigène exprimé par les cellules infectées par un virus, du fait de la réduction ou du blocage simultané d'une immunosuppression, d'une apoptose des cellules immunitaires ou d'une prolifération des cellules endothéliales vasculaires, ou encore l'induction d'une réponse immunitaire efficace et non délétère à l'encontre d'un allergène, du fait de la réduction ou du blocage de la commutation isotypique vers la production d'anticorps igE induite par l'IL4 ;
(ii) dans de nombreux cas, l'observation d'une potentialisation de la réponse immunitaire à l'encontre de l'un des polypeptides immunogènes présent dans le conjugué peptidique, du fait de la présentation conjointe du second polypeptide immunogène, porteur de nouveaux sites auxiliaires, aux cellules du système immunitaire, comme cela est observé par exemple dans le cas du superimmunogène composite gp160-Tat(toxoïde).

***Superimmunogènes composites utilisés pour le traitement de certains cancers et de certaines infections virales.***

Selon une première caractéristique essentielle d'un superimmunogène composite, le premier polypeptide (a) immunogène est choisi parmi (i) une protéine immunogène sélectivement exprimée par des cellules cancéreuses, sélectivement exprimée par des cellules infectées par un virus ou constitutive d'une structure antigénique pathogène allergène, le cas échéant détoxiquée, et (ii) une protéine dérivée de la protéine (i).

Selon une seconde caractéristique essentielle d'un superimmunogène composite, le polypeptide (b) immunogène est choisi parmi (i) la protéine circulante locale du stroma, le cas échéant détoxiquée, et (ii) une protéine dérivée de la protéine (i).

Lorsque la protéine immunogène sélectivement exprimée par des cellules cancéreuses, sélectivement exprimée par des cellules infectées par un virus ou constitutive d'une structure antigénique pathogène allergène est « toxique », par exemple « immunotoxique », ou lorsque la protéine circulante locale du stroma est « toxique », par exemple « immunotoxique », elle ne pourra être incluse telle quelle en tant que polypeptide (a) ou polypeptide (b) d'un superimmunogène composite, sans préalablement réduire ou bloquer sa toxicité, par exemple son immunotoxicité tout en conservant son immunogénicité.

La protéine immunogène est initialement « toxique » lorsque son administration à l'homme ou l'animal provoque des effets délétères importants sur la santé de cet homme ou de cet animal, par exemple soit en induisant une immunotoxicité paralysant ou déviant les réactions immunitaires de défense.

La protéine immunogène est initialement « immunotoxique » lorsque son administration à l'homme ou l'animal provoque l'une des effets suivants :

- une immunosuppression, y compris l'apoptose des cellules immunitaires, comme c'est le cas pour les protéines Tat de HIV1, E7 du papillomavirus, les facteurs cytokiniques IFNα, TGFβ ou IL10, ou encore le facteur de régulation cellulaire p53, sous sa forme circulante dans le stroma ;
- une déviation de la réaction immunitaire humorale, par exemple en induisant une commutation isotypique (« switch ») des lymphocytes B entraînant la production d'IgE., comme c'est le case de l'IL4.

La protéine immunogène initialement toxique, par exemple immunotoxique, peut être détoxiquée en la modifiant notamment physiquement, chimiquement ou par les techniques du génie génétique ; comme cela est exposé en détail plus loin dans la description.

Les techniques particulièrement chimiques qui permettent la détoxication d'un polypeptide (a) et/ou d'un polypeptide (b) peuvent aussi être mises en oeuvre pour la préparation d'un polypeptide (a) ou d'un polypeptide (b) à partir d'une protéine qui n'est pas initialement toxique ou immunotoxique, en raison de ce que ces traitements peuvent permettre

aussi de stabiliser le polypeptide pour une meilleure conservation du vaccin.

Il est essentiel que, tout comme les toxoïdes, toxines détoxiquées des vaccins anti-tétaniques ou anti-diphtériques, la protéine détoxiquée conserve son immunogénicité, c'est à dire sa capacité à induire :

- soit une réaction immunitaire cellulaire, ou cellulaire et humorale, à l'encontre de la structure antigénique pathogène, lorsque la protéine détoxiquée est préparée à partir de la protéine immunogène sélectivement exprimée par des cellules cancéreuses, sélectivement exprimée par des cellules infectées par un virus, ou constitutive d'une structure pathogène allergène ;
- soit une réaction immunitaire humorale à l'encontre de la protéine circulante locale du stroma telle que définie précédemment, lorsque la protéine détoxiquée est préparée à partir de cette protéine circulante du stroma.

Par « dérive » ou « dériver », d'une protéine immunogène contenue dans la structure antigénique pathogène ciblée ou de la protéine circulante locale du stroma ciblée, on entend que le polypeptide immunogènes (a) ou (b) peut être constitué d'un fragment peptidique inclus dans la protéine immunogène initiale ou bien que le polypeptide (a) ou (b) comprend une ou plusieurs substitutions, délétions ou additions d'acides aminés, par rapport à la séquence d'acides aminés de la protéine immunogène initiale, comme cela sera exposé plus loin dans la description. Dans tous les cas, un polypeptide (a) ou (b) qui « dérive » de la protéine immunogène initiale conserve sa capacité à induire une réaction immunitaire à l'encontre de la structure antigénique pathogène ciblée ou de la protéine circulante locale du stroma ciblée. Lorsque la protéine immunogène initiale est « toxique », le polypeptide (a) ou (b) qui en dérive possède une toxicité réduite ou a perdu sa toxicité, soit du fait qu'il ne constitue qu'un fragment non toxique de la protéine immunogène initiale, soit qu'il comporte des modifications d'acides aminés, par rapport à la séquence d'acides aminés de la protéine immunogène initiale, qui réduisent ou bloquent ses propriétés toxiques.

Selon un premier mode de réalisation d'un superimmunogène composite pour la prévention ou le traitement de certains cancers ou certaines infections virales, ledit superimmunogène composite est caractérisé en ce que le polypeptide (a) induit une réaction immunitaire cellulaire à l'encontre d'un antigène exprimé spécifiquement par les cellules cancéreuses ou par les cellules infectées par un virus.

Par « induction d'une réaction immunitaire cellulaire », on entend que le polypeptide (a), au sein de la structure du superimmonogène composite, est utile au déclenchement d'une réponse immunitaire efficace, laquelle, dans le cas des cellules cancéreuses et des cellules infectées par un virus, consiste principalement en la production soit de cellules T cytotoxiques (CTL) reconnaissant spécifiquement la structure antigénique pathogène visée, par exemple un antigène tumoral ou un antigène viral, qui est présentée à la surface de la cellule cancéreuse ou de la cellule infectée sous une forme associée aux antigènes de Classe I du complexe majeur d'histocompatibilité (MHC), soit de cellules tueuses NK, détruisant préférentiellement les cellules cancéreuses.

De plus, la réaction immunitaire cellulaire induite par le polypeptide immunogène (a) produit une activation des cellules T auxiliaires (« T helper») qui augmente ou permet d'induire la réaction humorale basée sur le polypeptide (b) immunogène et dirigée spécifiquement à l'encontre de la protéine circulante du stroma. Cela ne signifie pas pour autant qu'une réaction immunitaire humorale à l'encontre du polypeptide (a) n'est pas induite. Toutefois, l'induction d'une réponse humorale à l'encontre du polypeptide (a) dans le cas des cancers et des infections par un virus, si elle a lieu conjointement à l'induction de la réaction immunitaire cellulaire, n'est pas l'objectif poursuivi.

De préférence, l'antigène exprimé spécifiquement par les cellules cancéreuses du cancer du col utérin (HUCC) ou par les cellules infectées par le HIV ou par les cellules leucémiques ATL est choisi respectivement parmi (i) les protéines L1, L2 et E7 du papillomavirus, (ii) les protéines gp160, p24, p17, Nef et Tat du virus HIV-1, (iii) la protéine Tax, (iv) la protéine gp61 ou les protéines gag des virus HTLV1 ou 2, la protéine étant le cas échéant détoxiquée, ou encore une protéine qui en est dérivée.

Le polypeptide (a) est choisi de préférence parmi les protéines immunogènes du HIV1, des fragments immunogènes de ces protéines ou une protéine qui en est dérivée.

Préférentiellement, le premier polypeptide (a) immunogène est choisi parmi (i) les protéines L1, L2 et E7 du papillomavirus, (ii) les protéines gp160, p24, p17, Nef et Tat du virus HIV1, (iii) la protéine Tax des virus HTLV1 ou 2. le cas échéant détoxiquées ou une protéine qui en est dérivée.

Pour induire la réaction immunitaire à l'encontre de la structure antigènique pathogène, le polypeptide (a) peut consister en la protéine pathogène elle-même.

Certaines des protéines pathogènes listées ci-dessus possèdent des propriétés immunotoxiques, comme par exemple les protéines Tat du virus HIV1, E7 du papillomavirus ou Tax du virus HTLV1, et ne peuvent donc pas être utilisées telles quelles comme polypeptide (a) d'un superimmunogène composite.

Le polypeptide (a) sera dans ce cas choisi préférentiellement parmi (i) une forme inactivée, par exemple détoxiquée, de la protéine pathogène, (ii) un fragment inactif de la protéine pathogène et (iii) une protéine dérivée de la protéine pathogène, tel un mutant génétique, ou un fragment de cette protéine, dénués des propriétés initiales immunotoxiques.

Diverses techniques de préparation d'un polypeptide (a) tel que défini ci-dessus sont détaillées plus loin dans la description.

De préférence, le polypeptide (a) et le polypeptide (b) sont choisis parmi :

*a) Pour la prévention ou le traitement du SIDA :*

- Polypeptide (a) :les protéines gp160, p24, p17, nef, ou Tat du virus HIV1, détoxiquées ou stabilisées si cela est nécessaire, des fragments immunogènes de ces protéines ou encore une protéine immunogène qui en est dérivée (Zagury et al., 1998).
- Polypeptide (b) : les protéines Tat, IFNα, et TGFβ, détoxiquée si cela est nécessaire, des fragments immunogènes de ces protéines ou une protéine immunogène qui en est dérivée.

*b) Pour la prévention ou le traitement du cancer du col utérin :*

- Polypeptide (a): les protéines L1, L2 et E7 du papillomavirus, préférentiellement d'un papillomavirus de la souche 16 ou 18, détoxiquée ou stabilisée si cela est nécessaire, des fragments immunogènes de ces protéines ou encore une protéine immunogène qui en est dérivée (Le Buanec et al., 1999).
- Polypeptide (b): les protéines E7, IFNα, TGFβ, TNFα et VEGF, détoxiquées ou stabilisées si cela est nécessaire, des fragments immunogènes de ces protéines ou une protéine immunogène qui en est dérivée.

*c) Pour la prévention ou le traitement de la leucémie ATL induite par les virus HTLV1 ou 2:*

- Polypeptide (a) : les protéines gp61 et Tax des virus HTLV1 ou 2, détoxiquée si cela est nécessaire, des fragments immunogènes de ces protéines ou encore une protéine immunogène qui en est dérivée (Cowan et al., 1997 ; Mori et al., 1996).
- Polypeptide (b) : les protéines Tax, IL10, IFNα ou TGFβ, détoxiquées, des fragments immunogènes de ces protéines ou encore une protéine qui en est dérivée.

*d) Pour la prévention ou le traitement du cancer du colon :*

- Polypeptide (a) : les protéines CEA et p53, détoxiquées si cela est nécessaire, des fragments immunogènes de ces protéines ou encore un protéine immunogène qui en est dérivée (Zusman et al., (1996).
- Polypeptide (b): les protéines TGFβ, IL10, p53, FasL et VEGF, détoxiquées, des fragments peptidiques immunogènes de ces protéines ou encore une protéine immunogène qui en est dérivée.

*e) Pour la prévention ou le traitement du cancer du sein :*

- Polypeptide (a) :la protéine Di12, des fragments immunogènes de cette protéine ou encore une protéine qui en est dérivée (Yoshiji et al., 1996).
- Polypeptide (b) : les protéines TGFβ, TNFα et VEGF, détoxiquées si nécessaire, des fragments immunogènes de ces protéines ou encore une protéine immunogène qui en est dérivée.

*f) Pour la prévention ou le traitement du cancer du pancréas :*

- Polypeptide (a) :la protéine CaSm, détoxiquée si cela est nécessaire, des fragments immunogènes de cette protéine ou encore un protéine immunogène qui en est dérivée.
- Polypeptide (b) : les protéines VEGF et TNFα, détoxiquées ou stabilisées si nécessaire, des fragments immunogènes de ces protéines ou encore une protéine immunogène qui en est dérivée.

*g) Pour la prévention ou le traitement du cancer de la prostate :*

- Polypeptide (a) : les protéines OSA et ETS2, détoxiquées ou stabilisées si nécessaire, des fragments immunogènes de ces protéines ou encore une protéine immunogène qui en est dérivée. (Sementchenko VI et al., 1998).
- Polypeptide (b) : les protéines IL6 et TGFβ, détoxiquées ou stabilisées si nécessaire, des fragments immunogènes de ces protéines ou encore une protéine immunogène qui en est dérivée. (Adler et al., 1999).

Pour la prévention ou le traitement de certains autres cancers, on peut aussi utiliser, comme polypeptide

immunogène (a), les antigènes TSA (pour « Tumor Specific Antigen type ») ou les antigènes TAA (pour « Tumor Associated Antigen type »), des fragments immunogènes de ces protéines ou une protéine immunogène qui en est dérivée.

Les protéines gp160, Tat, Tax et p53 sont toxiques dans leur forme native. La préparation d'un polypeptide (a) immunogène à partir de ces protéines nécessitera impérativement de les détoxiquer, ou d'en préparer des fragments peptidiques immunogènes non toxiques ou encore une protéine non toxique qui en est dérivée, par exemple par un ou plusieurs substitutions, délétions ou additions d'acides aminés, par rapport à la séquence de la protéine native de référence.

Les protéines IFNα, Tat, TGFβ, E7, Tax, p53, et FasL, à concentration élevée, sont immunotoxiques, dans leur forme native. La préparation d'un polypeptide (b) immunogène à partir de ces protéines nécessitera impérativement de les détoxiquer, ou d'en préparer des fragments peptidiques immunogènes non toxiques ou encore une protéine non toxique qui en est dérivée, par exemple par un ou plusieurs substitutions, délétions ou additions d'acides aminés, par rapport à la séquence de la protéine native de référence.

La protéine immunogène VEGF pourra être utilisée, en tant que polypeptide (b) constitutif d'un superimmunogène composite, soit sous sa forme native, soit sous une forme stabilisée par traitement chimique, de préférence un traitement chimique d'un type utilisé pour détoxiquer les protéines.

Les polypeptides immunogènes (b), listés ci-dessus, et constituant un facteur cytokinique ou un facteur de régulation cellulaire à propriétés immunotoxiques ou angiogéniques, possèdent les caractéristiques détaillées ci-après.

- la protéine TGFβ : le TGFβ étant une cytokine immunosuppresive majeure produite par de nombreuses cellules cancéreuses ;
- la protéine IL10: l'IL 10 étant également une cytokine immunosuppresive majeure, ainsi que le FasL (Ligand Fas) ;
- la protéine p53 : la protéine de régulation p53 produite anormalement par les cellules cancéreuses peut représenter un antigène de tumeur associé (TAA) comme cela a été montré dans l'art antérieur. Lorsque la protéine p53 est libérée par les cellules anormales et s'accumule dans le compartiment stromal extracellulaire, elle agit alors comme facteur stromal immunosuppressif et apotogène (immunotoxique) sur les cellules immunitaires, et constitue en conséquence une protéine circulante stroale immunotoxique, au sens de l'invention, à l'encontre de laquelle il est recherché l'induction d'une réaction immunitaire humorale par un superimmunogène composite comprenant, en tant que polypeptide (b), la protéine p53 détoxiquée ou une protéine qui en dérive et qui a perdu les propriétés immunotoxiques de la protéine initiale.
- le VEGF, facteur de croissance des cellules endothéliales: la cytokine VEGF étant une cytokine majeure de l'angiogénèse, activant la prolifération des cellules endothéliales ;
- l'IL6, l'IFNγ et le TNFα, cytokines pro-inflammatoires participant également aux processus d'ongiogénèse, en activant l'expression des molécules d'adhérence des cellules endothéliales (ICAM, VCAM et E sélectine).

La réaction immunitaire humorale qui est induite par le polypeptide (b) constitutif d'un superimmunogène composite selon l'invention permet de rendre efficace la réaction immunitaire spécifique du polypeptide (a) :

(i) en neutralisant ou en bloquant l'activité immunosuppressive (l'immunotoxicité) de certaines protéines circulantes locales du stroma anormalement produites, comme l'IFNα ou la protéine Tat; ou

(ii) en neutralisant ou en bloquant les propriétés apoptogènes vis-à-vis des cellules immunitaires (l'immunotoxicité) induite par certaines protéines circulantes du stroma anormalement produites, comme le facteur de régulation cellulaire p53 ; ou

(iii) en neutralisant ou en bloquant l'angiogénèse, incluant la néovascularisation des tumeurs, induite par certaines protéines circulantes du stroma anormalement produites, comme le VEGF.

Une première famille préférée de superimmunogènes composites utilisés pour la prévention ou le traitement de certains cancers et de certaines infections virales est constituée des superimmunogènes composites suivants :

- le superimmunogène composite (a) gp160 - (b) Tat toxoïde ;
- le superimmunogène composite (b) peptide Tat [1-15 ;46-60] - (a) gp160 ;
- le superimmunogène composite (a) Tat toxoïde - (b) IFNα ;
- le superimmunogène composite (a) Tat toxoïde - (b) peptide Tat [1-15 ;46-60]

Une seconde famille préférée de superimmunogènes composites est constituée des superimmunogènes suivants :

- le superimmunogène composite (a) L1 - (b) E7 ;
- le superimmunogène composite (a) E7 - (b) VEGF ;

Ci-dessus, « (a) » désigne le polypeptide (a) et « (b) » désigne le polypeptide (b). La structure des superimmunogènes composites listés ci-dessus va, de la gauche vers la droite, de la partie $NH_2$-terminale vers la partie COOH-terminate.

Dans le superimmunogène composite (a) gp160 - (b) Tat toxoïde, la protéine gp160 induit une réaction immunitaire cellulaire à l'encontre des cellules infectées par VIH1, réaction immunitaire qui est facilitée par l'induction d'anticorps neutralisant ou bloquant les propriétés immunotoxiques de la protéine Tat de HIV1, connue pour provoquer la production d'IFNα, facteur cytokinique immunosupprssif.

Dans le superimmunogène (a) L1 - (b) E7, la protéine L1 de HPV induit une réaction immunitaire cellulaire à l'encontre des cellules infectées par HPV, qui peuvent être cancéreuses, réaction immunitaire qui est facilitée par l'induction d'anticorps neutralisant ou bloquant les propriétés immunotoxiques de la protéine E7 de HPV.

La protéine E7, qui est exprimée par la cellule cancéreuse du cancer du col utérin, laquelle, de ce fait, peut aussi être la cible de cellules T cytotoxiques ou de cellules NK reconnaissant la structure antigénique pathogène, peut aussi être utilisée, pour préparer un polypeptide (a) d'un superimmunogène composite, soit sous la forme de la protéine E7 détoxiquée, soit sous la forme d'une protéine dérivant de la protéine E7 et qui a perdu son immunotoxicité.

Dans le superimmunogène composite (a) E7 - (b) VEGF, la protéine E7 induit une réaction immunitaire cellulaire à l'encontre des cellules infectées par HPV, qui peuvent être cancéreuses, réaction immunitaire qui est rendue plus efficace du fait de l'induction d'anticorps neutralisant ou bloquant les propriétés angiogéniques du facteur cytokinique VEGF.

***Superimmunogènes composites utilisés pour la prévention ou le traitement des allergies.***

Dans un superimmunogène composite utilisé pour la prévention ou le traitement des allergies, le polypeptide (a) induit une réaction immunitaire humorale à l'encontre de la protéine pathogène allergène constitutive de la structure antigénique pathogène particulaire, qui peut, à titre illustratif, être un pollen, un acarien, une poussière ou un poil de chat.

De préférence la protéine pathogène allergène est choisie parmi les protéines Betv1a, Der p 1 et Fel d 1, bien connues de l'homme du métier

Préférentiellement, le polypeptide (a) est choisi parmi les protéines Betv1a, Der p 1 et Fel d 1, leurs fragments peptidiques immunogènes ou encore une protéine immunogène qui en est dérivée.

L'antigène Betv1 est décrit notamment par Ferreira et al. (1993), l'antigène Der p 1 est décrit notamment par Tovey et al. (1981) et l'antigène Fel d 1 est décrit notamment par Morgenstern et al. (1991)

Préférentiellement, le polypeptide (b), au sein du superimmunogène composite, induit la production d'anticorps neutralisants ou bloquants à l'encontre du facteur cytokinique IL4, qui est produit principalement par les lymphocytes T de type Th2, qui orientent la réponse immunitaire humorale vers la production d'anticorps d'isotype IgE.

Le polypeptide (b) est donc préférentiellement la protéine IL4 autologue, préférentiellement détoxiquée par traitement chimique, physique ou génétique, un fragment peptidique immunogène de cette protéine ou encore une protéine immunogène qui en est dérivée.

Selon un autre mode de réalisation, le polypeptide (b) induit la production d'anticorps neutralisants ou bloquants à l'encontre du facteur cytokinique IL5, qui est produit principalement par les lymphocytes T de type Th2.

Selon ce second mode de réalisation, le polypeptide (b) est préférentiellement la protéine IL5 autologue, préférentiellement détoxiquée, un fragment peptidique immunogène de cette protéine ou encore une protéine immunogène qui en est dérivée.

Dans le superimmunogène composite (a) Betv1a - (b) IL4 (souris), la protéine Betv1a induit une réaction immunitaire humorale, systémique ou mucosale, à l'encontre de la structure antigénique pathogène allergène par la production d'anticorps d'isotype IgG ou IgA, de préférence IgA sécrétoires, et la protéine IL4 induit une réaction immunitaire à l'encontre de ce facteur cytokinique, ce qui permet d'inhiber ou de bloquer la commutation isotypique vers la production d'anticorps IgE, à l'origine des symptomes allergiques, tout en induisant une réponse immunitaire efficace à l'encontre de l'allergène causal. La réaction immunitaire humorale obtenue à l'encontre de l'IL4, qui est une protéine autologue, est rendue possible grâce à la présence simultanée du polypeptide (a) également constitutif du superimmunogène composite, le polypeptide (a) comprenant des épitopes T auxiliaires qui induisent l'activation de cellules T auxilliaires nécessaires à la réaction immunitaire humorale des cellules B dirigées contre l'IL4.

Un « épitope T auxiliaire » est une région du polypeptide immunogène qui est reconnue sélectivement par le récepteur de l'antigène d'au moins un clone de lymphocytes T auxiliaires, l'événement de liaison du polypeptide immunogène au récepteur des lymphocytes T reconnaissant spécifiquement cette région d'épitope activant lesdits clones de lymphocytes T, qui vont se multiplier et produire des cytokines telles que l'IL2, qui vont à leur tour permettre le développement d'une réaction immunitaire humorale, y compris une réaction immunitaire humorale à l'encontre d'une protéine autologue, comme c'est le cas pour l'IL4 dans un superimmunogène composite selon l'invention.

***Inactivation des propriétés immunosuppressives ou apoptogènes d'un polypeptide (a) ou d'un polypeptide (b) constitutif d'un superimmunogène composite de l'invention, par détoxication.***

Comme exposé précédemment, un composé superimmunogène composite doit être dénué de toute toxicité et ne doit pas, en particulier du fait des propriétés intrinsèques de l'un de ces constituants, plus spécifiquement le polypeptide (a) ou le polypeptide (b), induire une suppression ou une déviation des réactions du système immunitaire visées.

Ainsi, lorsque le polypeptide (a) est un polypeptide initialement immunotoxique , celui-ci est tout d'abord rendu inactif pour ces propriétés avant d'être inclus dans un superimmunogène composite selon l'invention.

De même, lorsqu'un polypeptide (b) constitutif d'un conjugué peptidique immunogène est une cytokine ou un facteur de régulation cellulaire à propriétés immunotoxiques, soit immunosuppressives ou apoptogènes, soit susceptible de dévier la réaction immunitaire, par exemple en orientant la réaction des cellules T auxiliaires (« T helper ») vers la production de cellules de type Th2, comme c'est le cas de l'IL4, celui-ci est d'abord rendu inactif pour lesdites propriétés avant d'être inclus dans ledit superimmunogène composite.

Il est important d'utiliser le(s) polypeptide(s) (a) et/ou (b) délétère(s) sous forme notamment physiquement, chimiquement et/ou génétiquement modifiée (inactivée mais toujours immunogène) et non native (ou naturelle) ou sous une forme galénique appropriée afin qu'il n'exerce plus les effets immunotoxiques ou les propriétés de déviation de la réaction immunitaire de la protéine native, tout en conservant ses propriétés immunogènes.

En revanche, un polypeptide (a) ou un polypeptide (b) non immunotoxique, tel que le VEGF, peut être utilisé à l'état natif ou sous forme modifiée après un traitement de stabilisation..

Les traitements physiques peuvent être réalisés par la chaleur, les radiations U.V., les rayons X ou le contact avec une atmosphère riche en $O_2$. Ces traitements physiques générant des modifications intramoléculaires entre radicaux chimiques (groupement thiols par exemple), peuvent de manière appropriée changer la conformation de la molécule, l'inactiver fonctionnellement tout en conservant ses propriétés immunogènes.

Le traitement chimique peut être effectué à l'aide d'un agent de couplage tel qu'un dialdéhyde, ou d'une protéine porteuse activée par un prétraitement à l'aide d'un dialdéhyde, de préférence ou le glutaraldéhyde. Le traitement chimique peut se faire par utilisation d'un monoaldéhyde, notamment de formaldéhyde. On peut à cet égard se reporter à l'enseignement de WO-A-96/27389.

Le traitement chimique peut se faire notamment par d'autres procédés telle la carboxyméthylation ou la carboxamidation. Un exemple de technique de carboxyméthylation est illustré dans WO-A-99/33872. Le traitement chimique peut se faire également par N éthylmaléidation associée ou non à une glutaraldéhydation.

On peut encore citer comme technique d'inactivation la réaction d'au moins une fonction thiol de la protéine avec le 4-chloro-7-sulfobenzofurazane d'ammonium, le N-[iodoéthyl]-trifluoroacétamide ou le N-(6-[7-amino-4-méthylcoumarin-3-acétamido]hexyl)-3'-(2'-pyridyldithio)-propionamide ainsi que la réaction d'au moins fonction amino de la protéine avec l'éthylacétimidate, un anhydride, le 2-iminothiolane chlorhydrate, le N-succinimidyl S-acétylthioacétate, le sulfosuccinimidyl acétate, le sulfosuccinimidyl-4-O-[4,4-diméthoxytrityl]butyrate, le succinimidyl 7-amino-4-méthylcoumarin-3-acétate, le sulfosuccinimidyl 7-amino-4-méthylcoumarin-3-acétate ou le phénylglyoxal.

L'immunogène peut être inactivé grâce à une présentation galénique au sein d'un liquide huileux, tel l'adjuvant incomplet de Freund ou encore susceptible de modifier les liaisons non covalentes (forces électrostatiques, forces de Van der Waals ou liaisons hydrogène) nécessaires à ses effets toxiques.

Les modifications génétiques peuvent être obtenues par ingénierie génétique opérant des insertions, des délétions ou des substitutions de résidus, opérations destinées à diminuer ou supprimer les sites fonctionnels délétères de la molécule naturelle. Les mutants génétiques pourront ou non subir un traitement chimique et/ou physique complémentaire. Les protéines modifiées ci-dessus peuvent par exemple être préparées à partir d'une protéine ayant une séquence identique ou similaire à une séquence peptidique d'une protéine immunopathogène, en particulier immunosuppressive ou angiogénique, telle que la protéine Tat du VIH-1, la protéine E7 du papillomavirus ou la protéine Tax de HTLV1 ou d'un fragment de ces protéines et être obtenues par exemple par synthèse peptidique conventionnelle sur résine ou par génie génétique. Tous ces procédés sont bien connus de l'état de la technique. Les mutants inactifs mais immunogènes ont au moins une molécule d'ADN qui code pour leur production. De telles molécules d'ADN présentent un intérêt particulier dans la présente invention comme on le verra ci-après.

Afin de vérifier que la protéine native immunotoxique et/ou angiogénique est bien reconnue par des anticorps dirigés contre ladite protéine modifiée, détoxiquée ou stabilisée par traitement, ou son fragment modifié ou non selon l'invention, on peut par exemple vérifier immunologiquement par Elisa en présence d'anticorps spécifiques, la formation de complexes antigène-anticorps.

Dans des conditions préférentielles de mise en oeuvre, le composé immunogène provient d'un composé natif (protéine ou fragment poypeptidique) traité par un aldéhyde, ou carboxamidé, ou carboxyméthylé, ou maléimidé.

Afin de déterminer si les propriétés immunogènes de la protéine modifiée immunotoxique et/ou angiogénique ou d'un fragment de cette protéine ont été suffisamment conservées (c'est-à-dire si il ou elle a été inactivé(e) mais pas

dénaturé) pour créer des anticorps bloquant les effets de ladite protéine native, on peut par exemple immuniser des mammifères (lapins, rats, souris) à l'aide d'un composé immunogène selon l'invention et vérifier que les anticorps produits neutralisent les activités immunosuppressives, apoptogènes ou angiogéniques de la protéine.

Afin de déterminer si la protéine immunotoxique modifiée ou le fragment a perdu au moins la proportion désirée de ses propriétés immunotoxiques, on peut par exemple étudier l'effet de la protéine modifiée sur la production de cytokines Th2 (IL4) et Th1 (IFNγ) et/ou sur l'immunosuppression de cultures de cellules mononucléées du sang périphérique humain (PBMC) stimulées par des antigènes de rappel, etls que les antigènes PPD ou tétanos toxoïde.

La protéine immunotoxique (immunosuppressive ou apoptogène ou provoquant une déviation de la réaction immunitaire) ou angiogénique modifiée et immunogène peut dériver d'une quelconque des protéines notamment immunotoxiques à action locale induites par des cellules tumorales ou par des cellules infectées de malades du SIDA ; on retient particulièrement la protéine Tat du virus VIH-1, la protéine E7 du papilloma virus ou la protéine Tax du virus HTLV1. On retient aussi la lectine mannane-dépendante produite par des cellules immunitaires activées. On retient aussi le VEGF à propriétés angiogéniques et l'IL4 à propriétés immunotoxiques et provoquant une déviation de la réaction immunitaire vers une réponse Th2.

Comme déjà exposé précédemment, le polypeptide (a) ou le polypeptide (b) d'un superimmunogène composite peut consister en un fragment de la protéine immunogène initiale constitutive de la structure antigénique pathogène visée, ou encore en un polypeptide présentant, par rapport à la protéine immunogène initiale, des modifications dans les acides aminés.

Il peut comporter une ou plusieurs modifications dans les acides aminés de cette protéine ou fragment telles que des délétions, substitutions, additions, ou fonctionnalisations telles qu'acylation d'acides aminés, dans la mesure où ces modifications restent dans le cadre précisé ci-dessus (absence de toxicité, caractères immunologiques). Par exemple, en général le remplacement d'un résidu leucine par un résidu isoleucine ne modifie pas de telles propriétés ; les modifications doivent généralement concerner moins de 40% d'acides aminés, de préférence moins de 20% et tout particulièrement moins de 10% de la protéine immunopathogène, en particulier immunotoxique ou angiogénique. Il est important que la protéine ou fragment modifié ne soit pas dénaturé comme on peut le faire par exemple par un traitement physique comme la chaleur afin de préserver ses sites conformationnels pour que les anticorps induits par les dérivés modifiés soient actifs vis-à-vis de la protéine native.

Dans des conditions préférentielles, les composés immunogènes comportent au moins 50% de la totalité ou d'un segment de la protéine immunopathogène, en particulier immunotoxique ou angiogénique, de préférence au moins 70%, particulièrement au moins 90%, et tout particulièrement la totalité ou quasi-totalité de ladite protéine immunosuppressive ou angiogénique.

De manière générale, en ce qui concerne les modifications, l'homologie ou la similitude entre l'immunogène modifié et la protéine ou partie de protéine immunotoxique native, ainsi que les dimensions du polypeptide immunogène, de même que les modalités d'utilisation, ou de couplage du polypeptide immunogène constitutif d'un superimmunogène composite selon l'invention à une protéine immunogène telle que la toxoïde tétanique, on peut en particulier se référer à WO-A-86/06 414 ou à EP-A-0.220.273 ou encore à PCT/US 86/00831, équivalents, dont l'enseignement est incorporé ici par référence.

On préfère aussi un composé immunogène tel que défini ci-dessus qui est un produit obtenu par recombinaison génétique présentant une homologie peptidique de 70% au moins avec les protéines Tat du HIV-1, Tax du HTLV1 ou L2 et E7 de l'HPV ou la lectine mannane-dépendante produite par des cellules immunitaires activées ou avec un segment de ces protéines.

On préfère également un composé immunogène tel que défini ci-dessus caractérié en ce qu'il est traité par un aldéhyde, qu'il est carboxamidé, carboxyméthylé ou maléimidé.

On préfère enfin un composé immunogène tel que défini ci-dessus caractérisé par un conditionnement adjuvant qui le rend biologiquement inactif, telle qu'une émulsion huileuse en adjuvant de Freund incomplet (IFA).

On peut aussi faire dériver d'un mutant homologue le composé immunogène désiré.

Rappelons ici que par un conditionnement galénique d'une protéine active physiologiquement, on peut masquer son activité biologique tout en conservant son immunogénicité.

La réaction de carboxyméthylation permet de modifier les groupements thiols (groupements sulfhydryl) présents au niveau des résidus cystéines de l'enchaînement en acides aminés. La carboxyméthylation, technique bien connue de l'homme du métier, inactive certaines fonctions toxiques dépendantes des groupements SH selon la technique décrite pour la protéine Tat par Frankel et al.(Cell, vol.55 ,1988).

Mise à part la carboxyméthylation, la carboxamidation ou la maléimidation peuvent être utilisées pour bloquer les groupements SH et former des complexes S-carboxyéthylés, S-carboxamidés ou S-maléimidés.

Par exemple, la protéine Tat possède 7 cystéines. Ces systéines participent à la formation de ponts disulfure inter et intra-chaînes et contribuent à la formation d'oligomères.

Le produit de la réaction est dans chaque cas un résidu S-carboxyméthylcystéinyl ou S-carboxyméthylamidocystéinyl.

Un fragment peut comporter de 8 à 110 acides aminés par exemple, de préférence de 12 à 60 acides aminés et notamment de 12 à 40 acides aminés. Un tel fragment peut comporter aussi du ou des côtés C ou N terminal de 1 à 5 acides aminés supplémentaires c'est-à-dire différents du fragment d'origine. Un fragment doit en outre comporter une cystéine au moins pour pouvoir faire l'objet par exemple d'une carboxyméthylation. Les fragments, s'ils seront de préférence choisis inactifs par eux-mêmes, pourront en effet être soumis si désiré aux mêmes traitements d'inactivation que les protéines entières ou presque entières.

La réaction de carboxyméthylation ci-dessus peut aussi être réalisée avec d'autres agents chimiques comme l'acide performique, l'acide 3-bromopropionique, l'éthylénéimine, le bromure de (2-bromoéthyl)triméthylammonium, le 2-bromoéthane sulfonate, la 1,3-propanesulfone etc..

Dans des conditions préférentielles de mise en oeuvre du procédé ci-dessus décrit, ladite protéine ou ledit fragment de départ peut se présenter sous forme fusionnée à un marqueur (FP) ou non fusionnée (P). La forme FP peut modifier per se la conformation moléculaire et par là modifier son activité.

Les protéines ou fragments de départ du procédé sont des produits connus dont des procédés d'inactivation peuvent avoir été décrits dans la littérature comme dans WO-a-99/33872. Ces protéines de départ peuvent même être commercialisées (immunodiagnostics Inc. , Cat # 1002-2) ou peuvent être préparées de manière conventionnelle.

On peut en particulier préparer les protéines ou fragments de départ ci-dessus par :

1) Synthèse par génie génétique ou par synthèse biochimique ;
2) Purification.

Par génie génétique, on peut purifier les protéines produites par chromatographie d'affinité en utilisant par exemple des anticorps dirigés contre la protéine ou un de ses fragments ; on peut aussi synthétiser la protéine fusionnée à un marqueur (FP) qui servira à l'accrochage à une colonne d'affinité.

***Modes de réalisation préférés d'un superimmunogène composite de l'invention.***

Pour fabriquer un superimmunogène composite, on réalise un couplage du polypeptide (a) et du polypeptide (b) par voie chimique ou par recombinaison génétique.

Selon un mode de réalisation préférentiel du conjugué peptidique immunogène les polypeptides (a) et (b) sont inactivés dans leurs propriétés immunotoxiques et/ou stabilisées par formolation, carboxamidation, carboxyméthylation, maléimidation ou oxydation par barbotage d'oxygène.

Selon un autre aspect, les polypeptides (a) et (b), lorsqu'ils sont inactivés dans leurs propriétés immunotoxiques ou angiogéniques, sont inactivés par recombinaison génétique.

Dans un mode de réalisation particulier du conjugué peptidique immunogène, les polypeptides (a) et (b) sont liés directement entre eux de manière covalente, par exemple via une liaison peptidique-CO-NH-.

Cependant, afin d'introduire une certaine flexibilité dans la structure du conjugué peptidique immunogène, et notamment de permettre une mobilité dans l'espace des polypeptides (a) et (b), l'un par rapport à l'autre au sein du conjugué peptidique immunogène, on préfère un conjugué peptidique dans lequel les polypeptides (a) et (b) sont séparés l'un de l'autre, au sein dudit conjugué, par une chaîne espaceur.

Selon un premier mode de réalisation préféré d'un conjugué peptidique immunogène, les polypeptides (a) et (b) sont séparés l'un de l'autre, au sein dudit conjugué, par une chaîne espaceur choisie parmi le SMCC ou le SIAB, qui sont tous les deux des composés bifonctionnels.

Le composé SIAB, décrit par Hermanson G.T. (1996, Bioconjugate techniques, San Diego : Academic Press, pp 239-242), est le composé de formule (I) suivante :

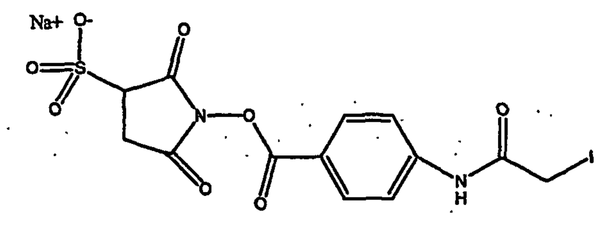

(I)

Le composé SIAB comprend deux groupes réactifs, respectivement un groupe iodoacétate et un groupe ester

Sulfo-NHS, ces groupes réagissant respectivement sur des groupes amino et sulfhydryl.

Le composé SMCC, qui est décrit par Samoszuk M.K. et al. (1989, Antibody, Immunoconjugates Radiopharm., 2(1) : 37-46), est le composé de formule (II) suivante :

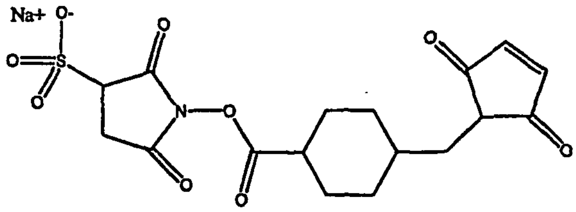

**(II)**

Le composé SMCC comprend deux groupes réactifs, respectivement un groupe ester Sulfo-NHS et un groupe maléimide, qui réagissent respectivement avec un groupe amino et un groupe sulfhydryl.

Selon un second mode de réalisation préféré, le superimmunogène composite comprend une chaîne espaceur constituée d'un peptide espaceur linéaire. On choisira de préférence un peptide espaceur linéaire ayant de 3 à 30 acides aminés de longueur, avantageusement de 5 à 20 acides aminés de longueur et de manière tout à fait préférée de 7 à 15 acides aminés de longueur.

Préférentiellement, le peptide espaceur linéaire est essentiellement, voire exclusivement, constitué d'acides aminés chargés positivement ou négativement à pH 7,0 afin d'accroître l'hydrophilicité globale dudit superimmunogène composite. On comprend que l'on devra éviter d'utiliser des peptides espaceurs constitués d'acides aminés hydrophobes. De manière préférentielle, le peptide espaceur est caractérisé en ce qu'il est constitué d'une chaîne de poly-(lysine) constituée de 3 à 30 résidus lysine, avantageusement de 5 à 20 et de manière tout à fait préférée de 7 à 15 résidus lysine de longueur.

Selon encore un autre mode de réalisation d'un superimmunogène composite, les polypeptides (a) et (b) sont séparés l'un de l'autre, au sein dudit conjugué peptidique, par une chaîne espaceur constituée d'un peptide espaceur ramifié, de préférence une structure oligodendrimérique de poly(lysine), telle que décrite par exemple par Basak et al. (1995).

Dans ce dernier mode de réalisation d'un superimmunogène composite, ledit conjugué peptidique peut comprendre plusieurs copies des polypeptides (a) et (b) par molécule de conjugué, avantageusement de 2 à 8 copies des polypeptides (a) et (b), de préférence au plus 4 copies de chacun des polypeptides (a) et (b) par molécule de conjugué.

Les polypeptides (a) et (b) peuvent aussi être inclus tous les deux au sein d'une même structure physique par exemple sur des monoparticules ayant un diamètre compris entre 10 et 500 nanomètres de préférence 10 et 1000 nanomètres, et de manière tout à fait préférée entre 10 et 100 nanomètres, par exemple des nanopaticules d'IMS, comme décrit par exemple par Aucouturier et al. (2001), de chitosan , comme décrit par exemple par Sjaugrud et al. (1999), de liposomes, ou de particules biodégradables comme le polylactide acide (PLA), le poly-ε-caprolactone (PLC) ou le poly(lactide-coglycolide) (PLG) décrit par Baras et al. (1999).

Selon encore un autre mode de réalisation, les polypeptides (a) et (b) sont liés physiquement au sein d'une même structure porteuse permettant leur présentation simultanée aux cellules du système immunitaire. Dans ce mode de réalisation particulier, les polypeptides (a) et (b) sont immobilisés sur des nanoparticules, par exemple des nanoparticules de chitosan, de IMS (Immunomodulateur accessible auprès de la Société Seppic, France), constitué de nanoparticules lipidiques d'un diamètre de 100 à 300 nanomètres) ou de liposomes.

Préférentiellement, de telles nanoparticules sont de faible taille de manière à présenter simultanément les polypeptides (a) et (b) aux cellules, de la même manière que si ces polypeptides étaient liés de manière covalente au sein de la même molécule. Avantageusement les nanoparticules ont un diamètre compris entre 10 et 1000 nm, mieux entre 10 et 500 nm, préférentiellement entre 10 et 300 nm et de manière tout à fait préférée entre 10 et 200 nm.

L'utilisation d'un conjugué peptidique immunogène tel que défini ci-dessus trouve une application majeure pour le traitement du SIDA, des cancers et de l'allergie.

La présente description a encore pour objet un composé superimmunogène composite comprenant deux polypeptides immunogènes distincts physiquement liés l'un à l'autre, les deux polypeptides consistant respectivement en :

(a) un premier polypeptide immunogène induisant une réaction immunitaire cellulaire, ou une réaction immunitaire cellulaire et humorale, à l'encontre d'une structure antigénique pathogène cellulaire, microbienne ou particulaire inerte ou vivante ;

(b) un second polypeptide immunogène induisant la production d'anticorps neutralisants ou bloquants à l'encontre d'une protéine circulante locale du stroma choisie parmi un facteur cytokinique ou un facteur de régulation cellulaire à propriétés immunotoxiques ou angiogéniques, ce facteur pouvant être soit produit par les cellules cancéreuses, les cellules infectées par un virus ou les cellules stromales, y compris les lymphocytes T et les cellules présentant l'antigène (APC), soit induit par les structures particulaires pathogènes, notamment allergéniques.

Les caractéristiques détaillées du superimmunogène composite ci-dessus ont déjà été exposées précédemment dans la présente description, auquel l'homme du métier pourra se référer.

Dans un premier mode de réalisation préférentiel, le conjugué peptidique immunogène est caractérisé en ce qu'il consiste en le conjugué gp160 - Tat toxoïde.

Selon un second mode de réalisation préférentiel, le conjugué peptidique est caractérisé en ce qu'il consiste en en le conjugué Tat toxoïde-IFN$\alpha$.

Selon un troisième mode de réalisation préférentiel, le conjugué peptidique immunogène est caractérisé en ce qu'il consiste en E7-SIAB-VEGF.

Selon un quatrième mode de réalisation préférentiel, le conjugué peptidique immunogène est caractérisé en ce qu'il consiste en BetV1a-SIAB-IL-4.

***Utilisation d'un acide nucléique codant un superimmunogène composite***

La description a aussi pour objet un acide nucléique codant certains des composés superimmunogènes composites, c'est à dire les superimmunogènes peptidiques constitués exclusivement d'une seule chaîne peptidique linéaire comprenant les polypeptides (a) et (b) liés entre eux, soit directement, soit via une chaîne espaceur peptidique linéaire, telle que définie ci-dessus.

Elle est également relative à une cassette d'expression fonctionnelle chez un mammifère, de préférence chez l'homme, comprenant un acide nucléique tel que défini ci-dessus, qui est placé sous le contrôle d'un polynucléotide régulateur fonctionnel chez un mammifère, de préférence chez l'homme.

Elle concerne aussi un vecteur recombinant comprenant un acide nucléique ou une cassette d'expression tels que définis ci-dessus, permettant l'expression d'un superimmunogène composite selon l'invention chez un mammifère, de préférence chez l'homme.

Elle a également trait à l'utilisation d'un acide nucléique, d'une cassette d'expression ou d'un vecteur recombinants tels que définis ci-dessus pour l'obtention d'un médicament à action anti-cancéreuse, anti-virale ou anti-allergique.

De manière générale, l'ADN (plasmide avec promoteur) peut être délivré aux surfaces mucosales sous forme d'ADN nu ou formulé, par exemple sous forme de liposomes cationiques ou concentré autour de particules d'or ou encore sous forme de microsphères. Il est avantageusement mis en oeuvre en présence d'adjuvants notamment des toxines bactériennes telles que CTB (cholera toxine) ou de LT (enterotoxine labile d'E. coli), de préférence mutée ($LT\mu$). De telles techniques d'immunisation mucosale avec des vaccins à base de molécules d'ADN sont décrites notamment dans Microbes and Infection, 1999, 685-698 par McCluskie et al.

Selon un mode de réalisation avantageux, un vecteur recombinant selon l'invention comprendra notamment les éléments suivants :

(1) des éléments de régulation de l'expression de l'acide nucléique codant le superimmunogène composite, tels que des promoteurs et des séquences activatrices (" enhancers ") ;

(2) la séquence codante comprise dans l'acide nucléique codant un superimmunogène composite à insérer dans un tel vecteur, ladite séquence codante étant placée en phase avec les signaux de régulation décrits aux (1) ; et

(3) des séquences d'initiation et d'arrêt de la transcription appropriées.

En outre, les vecteurs recombinants pourront inclure une ou plusieurs origines de réplication chez les hôtes cellulaires dans lesquels leur amplification ou leur expression est recherchée, des marqueurs ou des marqueurs de sélection.

Un vecteur recombinant peut aussi être un vecteur rétroviral ou encore un vecteur adéno-associé (AAV). De tels vecteurs adéno-associés sont par exemple décrits par Flotte et al. (1992), Samulski et al. (1989), ou encore McLaughlin BA et al. (1996).

Pour introduire les polynucléotides ou les vecteurs dans une cellule hôte, l'homme du métier pourra avantageusement se référer à différentes techniques, comme la technique de précipitation au phosphate de calcium (Graham et al., 1973 ; Chen et al., 1987), le DEAE Dextran (Gopal, 1985), l'électroporation (Tur-Kaspa, 1896 ; Potter et al., 1984), la microinjection directe (Harland et al., 1985), Les liposomes chargés en ADN (Nicolau et al., 1982, Fraley et al., 1979).

Selon un mode de réalisation particulier, une méthode pour introduire un polynucléotide dans une cellule hôte,

en particulier une cellule hôte provenant d'un mammifère, *in vivo,* comprend une étape au cours de laquelle on introduit une préparation comprenant un vecteur pharmaceutiquement compatible et un polynucléotide " nu ", placé sous le contrôle de séquences de régulation appropriées, par injection locale au niveau du tissus choisi, par exemple un tissu musculaire lisse, le polynucléotide " nu " étant absorbé par les cellules de ce tissu.

Des compositions pour l'utilisation *in vitro* et *in vivo* comprenant des polynucléotides " nus " sont par exemples décrites dans la demande PCT N° WO 95/11307 (Institut Pasteur, Inserm, Université d'Ottawa) ainsi que dans les articles de Tacson et al. (1996) et de Huygen et al. (1996).

Il peut encore s'agir de vecteurs adénoviraux tels que l'adénovirus humain de type 2 ou 5.

La quantité de vecteur qui est injecté à l'organisme hôte choisi varie selon le site de l'injection. A titre indicatif, il peut être injecté entre environ 10 μg et 1000 μg du polynucléotide codant un superimmunogène composite dans le corps d'un animal, de préférence d'un patient.

**Compositions immunogènes ou vaccinales comprenant un superimmunogène composite ou un acide nucléique codant celui-ci.**

La description a encore pour objet une composition immunogène caractérisée en ce qu'elle comprend, à titre de principe actif, un superimmunogène composite: tel que défini : ci-dessus, en association avec un ou plusieurs excipients physiologiquement compatibles.

Elle concerne une composition immunogène caractérisée en ce qu'elle comprend une quantité efficace, par exemple immunologiquement active, d'un superimmunogène composite tel que défini dans la présente description.

Selon les objectifs poursuivis, on utilise des adjuvants systémiques ou des adjuvants mucosals. Par exemple, on utilise de préférence un adjuvant mucosal pour prévenir les cancers des tissus épithéliaux et on utilise de préférence des adjuvants systémiques pour prévenir ou traiter les infections par des virus, tels que par HIV1 et HTLV1, ou encore pour prévenir ou traiter les allergies.

Parmi les adjuvants systémiques, on utilise de préférence les adjuvants de type IFA (Adjuvant Incomplet de Freund), le phosphate de calcium ou l'hydroxyde d'alumine.

Parmi les adjuvants mucosals, on utilise de préférence des adjuvants comme la chloratoxine B (CTB) ou un mutant de la toxine LT (LTμ).

Elle a également trait à un vaccin, mucosal ou systémique, caractérisé en ce qu'il comprend, à titre de principe actif, un superimmunogène composite tel que défini ci-dessus, en association avec un ou plusieurs excipients, dont des adjuvants d'immunité, physiologiquement compatibles.

Les compositions immunogènes ou les vaccins trouvent leur emploi par exemple dans le traitement tant curatif que des cancers, notamment des cancers induits par des virus comme par exemple, l'ATL (Acute T cell leukemia) causé par le HTLV1, ou le cancer du col utérin causé par le papilloma virus, ou encore le lymphome de Burkitt ou le sarcome de Kaposi causés par des virus de la famille herpès, respectivement l'Epstein-Barr (EBV) et le HHV8 ainsi que dans le traitement du SIDA, ainsi que pour prévenir ou traiter les réactions inflammatoires allergiques.

Les superimmunogènes composites peuvent être utilisés comme suit :

On administre à un patient, sous une forme adaptée à l'administration systémique ou mucosale, un composé superimmunogène composite ou une molécule d'ADN, par exemple par voie intranasale, en quantité suffisante pour être efficace sur le plan thérapeutique, à un sujet ayant besoin d'un tel traitement. La dose administrée peut aller par exemple de 10 à 1000 μg par voie intranasale, une fois par semaine pendant deux mois, puis périodiquement en fonction du taux des anticorps sécrétoires induits, par exemple tous les 2-6 mois.

On pourra administrer dans une même préparation deux ou plusieurs molécules superimmunogènes composites et/ou molécules d'ADN différentes pour induire des anticorps neutralisant tous les sites fonctionnels délétères au cas ou une seule molécule ne porte pas tous les sites actifs de la toxine ou de la cytokine surproduite que l'on veut neutraliser.

L'invention a aussi pour objet les compositions pharmaceutiques destinées aux muqueuses qui renferment au moins un conjugué peptidique immunogène ou molécule d'ADN précité, à titre de principe actif.

A titre de médicaments, les conjugués peptidiques immunogènes ou molécules d'ADN de l'invention peuvent être incorporés dans des compositions pharmaceutiques destinées à une administration par voie systémique ou bien à une administration par la voie muqueuse, notamment oro-muqueuse, en particulier la voie intranasale, la voie orale et la voie vaginale. L'administration peut avoir lieu en dose unique ou répétée une ou plusieurs fois après un certain intervalle de temps.

C'est pourquoi la présente demande a également pour objet une composition pharmaceutique, curative ou préventive, caractérisée en ce qu'elle comprend à titre de principe actif, un ou plusieurs superimmunogènes composites tels que définis ci-dessus, ou ses fragments ou molécules d'ADN correspondant à la protéine native à combattre. Le composé immunogène, fragment ou molécule d'ADN peut être conditionné seul ou mélangé à un excipient ou mélange d'excipients pharmaceutiquement acceptables tel qu'un adjuvant. Parmi les excipients destinés à la voie intranasale ou

orale, on retient particulièrement les capryl caproyl macrogol glycérides comme le Labrasol® de la Société GATTEFOSSE ou l'hydroxyde d'alumine (Alhydragel, Superfos, Danemark).

Il est à noter qu'administré tel quel, selon une formulation orale conventionnelle, le principe actif selon l'invention serait inactif.

Pour l'administration orale, on peut associer le principe actif à un adjuvant d'immunité mucosale tel qu'un mutant de CT, de LT ou de CTB.

On retient tout particulièrement les formes galéniques décrites par Boyaka et al. « Strategies for mucosal vaccine development » dans Am. J. Trop. Med. Hyg. 60(4), 1999, pages 35-45. On peut citer aussi les microgranules gastro résistants, notamment bioadhésifs tells que ceux décrits par Rojas et al dans Pharmaceutical Research, vol. 16, n°2, 1999, page 255.

Dans des conditions particulières de mise en oeuvre, on retient une composition pharmaceutique vaccinale ci-dessus, caractérisée en ce qu'elle comprend un adjuvant d'immunité mucosale, tel un mutant de CT (cholera toxine) ou de LT (entérotoxine labile d'*E. coli*).

Dans d'autres conditions particulières de mise en oeuvre, on retient une composition pharmaceutique vaccinale ci-dessus, caractérisée en ce qu'elle renferme un adjuvant absorbant le principe actif, tels l'hydroxyde d'alumine où les particules d'or.

Dans encore d'autres conditions préférentielles de mise en oeuvre, on retient une composition pharmaceutique ci-dessus, caractérisée en ce que le conjugué peptidique immunogène est obtenu par recombinaison génétique.

Dans toujours d'autres conditions, préférentielles de mise en oeuvre, on retient une composition pharmaceutique ci-dessus, caractérisée en ce que le polypeptide (a) et/ou le polypeptide (b) constitutif du conjugué peptidique a été traité par un aldéhyde a été carboxyméthylé, carboxamidé ou maléimidé.

La présente description a encore pour objet un procédé de préparation d'une composition ci-dessus décrite, caractérisé en ce que l'on mélange, selon des méthodes connues en elles-mêmes, le ou les principes actifs avec des excipients acceptables, notamment pharmaceutiquement acceptables et le cas échéant, avec un adjuvant d'immunité systémique ou mucosale.

Dans des conditions préférentielles de mise en oeuvre du procédé ci-dessus, on prépare des microgranules bioadhésifs et gastro résistants pour la voie orale digestive renfermant les principes actifs immunogènes et le cas échéant les adjuvants.

La description a aussi pour objet une composition immunogène caractérisée en ce qu'elle comprend une quantité thérapeutiquement efficace d'un acide nucléique codant un superimmunogène composite tel que défini dans la présente description, une cassette d'expression comprenant un tel acide nucléique, ou un vecteur recombinant comprenant cet acide nucléique ou cette cassette d'expression.

Elle a également trait à un vaccin caractérisé en ce qu'il comprend une quantité thérapeutiquement active d'un acide nucléique, d'une cassette d'expression ou d'un vecteur recombinant tels que définis ci-dessus.

La présente invention est en outre illustrée par les exemples suivants :

## EXEMPLES

### Exemples de préparations de superimmunogènes composites

### Exemple 1 : Préparation d'un conjugué [gp160 - Tat Toxoïde]

Ce conjugué doit représenter le principe actif d'un vaccin composite apte à induire principalement chez le vacciné une réaction cellulaire (chimiokines ; T auxiliaire, CTL) dirigée contre les cellules infectées exprimant la gp160 et une réaction anticorps contre la protéine Tat extracellulaire.

810 μg de la protéine gp160 dissous dans 1 ml de PBS ont été activés par dialyse, pendant une nuit, contre 100 ml d'une solution de glutaraldéhyde à 0,2 % dans du PBS, après quoi, l'excès de glutaraldéhyde a été éliminé par 3 dialyses successives contre 100 ml de PBS, de 2 heures chacune.

A 1 ml de gp160 ainsi activée ont été ajoutés 522 μl d'une solution de Tat toxoïde à 1,55 ml/ml (soit 810 μg de Tat Toxoïde). Le mélange a été agité, pendant 1 nuit, à 4° C, puis la réaction bloquée par addition de 50 μl d'une solution de glycine 2,5 M. Le mélange réactionnel a été finalement purifié par chromatographie d'exclusion. L'antigénicité de la préparation vis-à-vis de la protéine Tat et de la protéine gp160 correspond à l'antigénicité de chacune des protéines déterminées isolément.

### Exemple 2 : Préparation d'un conjugué [Tat Toxoïde - IFNα]

Il s'agit de la production d'un vaccin composite propre à induire principalement une réaction immunitaire cellulaire (chimiokines ; T auxiliaire, CTL) dirigée contre les cellules infectées exprimant la protéine Tat et une réaction

immunitaire humorale dirigée contre l'IFNα. De plus ce conjugué pourra induire la formation d'anticorps dirigés contre la protéine Tat extracellulaire.

Le couplage a été réalisé par réaction de l' IFNα réduit avec la molécule Tat Toxoïde activée par le SIAB (cf. ex. 2).

1. Activation du Tat Toxoïde (Tx)

5 mg de Tx dissous dans 3 ml de PBS-EDTA 5mM ont été additionnés de 187 μl d'une solution de SIAB (1,7 mg/ml). Après une heure de réaction à la température du laboratoire, le mélange réactionnel a été filtré à travers une colonne de Sephadex G25 équilibrée en tampon borate 0,1 M-EDTA 5 mM, pH 8,5.

2. Réduction de l'IFNα

Mêmes conditions que dans l'exemple 3. Récupération de 2,6 mg d'IFNα réduit.

5 mg de Tx-SIAB ont été mélangés, sous agitation avec 2,6 mg d'IFNα réduit et l'agitation a été poursuivie à la température du laboratoire pendant 1 heure puis pendant 1 nuit à 4° C. La réaction a été bloquée par addition de Cys,HCl à concentration finale 5 mM et réaction pendant 30 min.

Le mélange réactionnel a été purifié par chromatographie d'exclusion. L'antigénicité du Tat-Toxoide et de l'IFNα s'est révélée conservée sans le conjugué.

## Exemple 3 : Préparation d'un conjugué [Tat peptides (1-15) (46 - 60) SIAB-Tat]

Il s'agit de la production d'un vaccin composite propre à induire principalement une réaction immunitaire cellulaire (chimiokines ; T auxiliaire, CTL) dirigée contre les cellules infectées exprimant la protéine Tat et une réaction immunitaire humorale dirigée contre la protéine Tat extracellulaire.

1. Activation des peptides Tat avec SIAB

Un mélange de 1,5 mg de chacun des peptides (1 - 15 et 46 - 60) dissous dans 600 μl d'eau ont été activés par traitement, pendant 1 heure, à la température du laboratoire avec 500 μl d'une solution de SIAB à 1,7 mg/ml dans PBS, après quoi le mélange réactionnel a été filtré sur Sephadex G25 (colonne 1 x 15 cm) équilibrée en tampon PBS.

2. Réduction de la protéine Tat

Tat réduit par 2 mercaptoethylamine (cf. conditions dans exemple 3).

3. Couplage de la protéine Tat aux peptides Tat

0,75 mg de Tat peptides activés par SIAB ont été mélangés avec 1,25 mg de Tat réduit, et le mélange a été agité pendant 45 min. à la température du laboratoire, puis conservé pendant 1 nuit à 4° C. La réaction a été bloquée par addition de Cys, HCl à concentration finale 5 mM, puis le mélange a été purifié filtration à travers des membranes calibrées. La protéine Tat dans le conjugué a été inactivée par carboxamidation.

L'antigénicité du conjugué Tat peptides - Tat toxoïde par rapport à celle du Tat toxoïde a été déterminée à l'aide d'un ELISA indirect classique. Le conjugué Tat peptides-Tat toxdide présente une antigénicité égale à l'antigénicité de la protéine Tat toxoïde.

## Exemple 4 : Préparation d'un conjugué [E7-SIAB-VEGF)

Ce conjugué représente le principe actif d'un vaccin composite propre à induire principalement chez le vacciné une réaction cellulaire (chimiokines ;T auxiliaire, CTL) dirigée contre les cellules infectées exprimant la protéine E7 et une réaction anticorps contre la protéine VEGF. De plus ce conjugué pourra induire la formation d'anticorps dirigés contre la protéine E7 extracellulaire.

1. Activation de la protéine E7

1 mg de protéine E7 est dissous dans 1 ml de tampon borate 0,1 M - EDTA 5 mM, pH 8,5, contenant 20 % de dioxane. A cette solution sont ajoutés 400 μl d'une solution de SIAB à 1,7 mg/ml dissous dans le même tampon. La réaction est poursuivie pendant 1 heure à la température du laboratoire et le mélange réactionnel est appliqué à une colonne de Sephadex G25 (1 x 16 cm) équilibrée avec le tampon borate-EDTA-dioxane et les fractions correspondant

à la protéine sont recueillies et poolées.

2. Réduction de la protéine VEGF

La réduction a été effectuée par réaction avec la 2-mercaptoethylamine dans les conditions décrites à l'exemple 3.

3. Couplage de VEGF réduit à E7-SIAB

1 mg de VEGF réduit a été mis à réagir avec 1 mg de E7 activé par le sulfo-SIAB. Après 1 heure de réaction à la température du laboratoire, la réaction a été bloquée par addition de cystéine à concentration finale 5 mM et le mélange réactionnel concentré par diffusion contre du saccharose solide. La solution concentrée a été finalement purifiée par chromatographie d'exclusion. L'antigénicité du E7 et de VEGF est conservée dans le conjugué.

**Exemple 5 : Préparation d'un conjugué [Bet V 1a - SIAB - Il-4 (murin)]**

Ce conjugué représente le principe actif d'un vaccin composite destiné à orienter la réponse vers la formation d'anticorps de classe IgG et non plus IgE contre l'allergène du bouleau Bet V 1a.

1. Réduction de l'Il-4

1 mg de Il-4 dissous en 250 µl de tampon phosphate 10 mM, pH 7,2 + EDTA 1 mM a été réduit par la 2-mercaptoéthylamine conformément aux conditions de l'exemple 3 et la cytokine réduite recueillie par gélification sur Sephadex G25.

2. Activation de la protéine Bet V 1a par SIAB

1 mg de la protéine Bet V 1a dissous dans 500 µl de tampon phosphate 10 mM - EDTA 1 mM a été diluée par 80 µl de solution à 1,7 mg/ml de sulfo-SIAB pendant 2 heures à température du laboratoire. Le mélange réactionnel a été purifié par chromatographie à exclusion.

3. Couplage de l'IL4 réduit à Bet V 1a-SIAB

1 mg d'IL4 réduit a été mis à réagir avec 0,5 mg de Bet V 1a activé par le sulfo-SIAB. Après 1 heure de réaction à la température du laboratoire, la réaction a été bloquée par addition de cystéine à concentration finale 5 mM et le mélange réactionnel concentré par diffusion contre du saccharose solide. La solution concentrée a été finalement purifiée par chromatographie d'exclusion. L'antigénicité de l'IL4 et du Bet V 1a est conservée dans le conjugué.

**Exemples d'activité immunogénique des superimmunogéniques**

**Exemple 6 : Activité immunogénique du conjugué gp160-Tat toxoïde**

***A. Matériel et méthodes***

L'activité immunogénique de la préparation gp160-Tat toxoïde par rapport à celle du Tat toxoïde seul et son absence de toxicité ont été déterminées chez la souris Balb c de 18-20 g.

*1-Immunisation :*

Au jour 0, un groupe de 3 souris reçoit une injection de 0.2 ml (50 µg) d'une émulsion en ACF par voie intramusculaire. Une injection de rappel de 5 µg en AIF est donnée à J21 et J60.
Un prélèvement sanguin au niveau rétro-orbital est effectué sur chaque souris avant la première injection à J-2
3 souris contrôles reçoivent les mêmes préparations sans immunogène.
Les souris sont sacrifiées 12 jours après la dernière immunisation.

2-Toxicité :

La toxicité anormale est recherchée chez 3 souris recevant 1 dose humaine (100 µg) selon la pharmacopée.

L'absence d'immunotoxicité du superimmunogène est évaluée in vitro par un test de prolifération cellulaires réalisé sur des PBMCs cultivés en présence du conjugué et stimulées par du PPD ou du Tétanos, toxoïde.

***B. Résultats :***

1-Absence de toxicité du superimmunogène in vivo et in vitro

Les souris immunisées aussi bien par la préparation de gp160-Tat toxoïde que par le Tat toxoïde uniquement ne présentent aucun signe clinique et aucune lésion anatomique. Le test d'immunosuppression montre que des doses de 100 ng/ml à 3 μg/ml de gp160-Tat toxoïde ne diminuent pas la prolifération des lymphocytes.

Aucune des 3 souris immunisées avec 100 μg de la préparation ne manifeste de signes de toxicité (température, troubles cutanés, manifestations systémiques ou régionales) pendant les 7 jours suivant l'injection.

**2- Réponse humorale**

La réponse humorale est mesurée par la présence dans le sérum d'anticorps de type IgG dirigée contre la protéine recombinant Tat native, mesurée par ELISA et exprimée en titre (inverse de la dilution donnant une densité optique supérieure à 0.3)

**TABLEAU 1**

| | Titre | |
|---|---|---|
| | j-2 | J72 |
| souris contrôle : | | |
| souris contrôle 1 | $<500^{-1}$ | $<500^{-1}$ |
| souris contrôle 2 | $<500^{-1}$ | $<500^{-1}$ |
| souris contrôle 3 | $<500^{-1}$ | $<500^{-1}$ |
| souris immunisée avec le Tat toxoïde : | | |
| souris 4 | $<500^{-1}$ | $32\ 000^{-1}$ |
| souris 5 | $<500^{-1}$ | $48\ 000^{-1}$ |
| souris 6 | $<500^{-1}$ | $48\ 000^{-1}$ |
| souris immunisée avec le conjugué gp160-Tat toxoïde : | | |
| souris 7 | $<500^{-1}$ | $64\ 000^{-1}$ |
| souris 8 | $<500^{-1}$ | $>64\ 000^{-1}$ |
| souris 9 | $<500^{-1}$ | $>64\ 000^{-1}$ |

Les souris immunisées avec la préparation de gp160-Tat toxoïde présentent des titres d'anticorps de type IgG anti-Tat plus importants que ceux des souris immunisées avec le Tat toxoïde uniquement.

L'activité neutralisante de ces anticorps a été mesurée à l'aide du Cat assay. Différentes dilutions de sérums (1/50- 1/400) prélevés à J-2 et J72 sont incubées pendant 2 heures avec 50 ng/ml de Tat natif. Ces dilutions sont ensuite déposées sur des cellules HeLa, cellules transfectées de manière stable avec un plasmide contenant le LTR du VIH-1 comme promoteur du gène Chloramphénicol Acétyl transférase (CAT). Après 24 heures de culture, les cellules sont lysées et la quantité de protéine CAT produite est mesurée par un test ELISA, le Cat assay, (Boehringer Mannheim). Les sérums neutralisants empêchent la protéine Tat d'induire l'expression de la protéine CAT, alors que les sérums non neutralisants permettent la synthèse de cette protéine CAT. Les résultats sont données en % de neutralisation.

**TABLEAU 2**

souris immunisée avec le Tat toxoïde :

| | 1/50 | 1/100 | 1/200 | 1/400 |
|---|---|---|---|---|
| souris 4 j-2 | 0 | 0 | 0 | 0 |
| j72 | 75 | 50 | 25 | 20 |
| souris 5 j-2 | 0 | 0 | 0 | 0 |

(suite)

souris immunisée avec le Tat toxoïde :

| | 1/50 | 1/100 | 1/200 | 1/400 |
|---|---|---|---|---|
| j72 | 75 | 60 | 30 | 20 |
| souris 6 j-2 | 0 | 0 | 0 | 0 |
| j72 | 75 | 60 | 30 | 20 |

**TABLEAU 3**

| | 1/50 | 1/100 | 1/200 | 1/400 |
|---|---|---|---|---|
| souris 7 j-2 | 0 | 0 | 0 | 0 |
| j72 | 100 | 100 | 100 | 75 |
| souris 8 j-2 | 0 | 0 | 0 | 0 |
| j72 | 100 | 100 | 100 | 100 |
| souris 9 j-2 | 0 | 0 | 0 | 0 |
| j72 | 100 | 100 | 100 | 100 |

Les anticorps induits par le conjugué gp160-Tat toxoïde ont un pouvoir neutralisant plus important que celui induit par le Tat toxoïde.

## 3. Réponse cellulaire

### 3.1 Production de MP1α ET DE MP1β dans les surnageants de culture des splénocytes

Les splénocytes des souris immunisées et des souris contrôles sont isolées puis cultivées dans des puits à fond rond d'une plaque de micro-culture à raison de 100 000 cellules/puits en présence de 5 μg/ml de p24, de gp160, de Tat natif et d'un mélange de 5 μg/ml gp160 et de 5 μg/ml de Tat natif. Les sumageants sont prélevés après 24 heures de culture et la présence de MIP1α et MIP1β dans les sumageants est mesurée à l'aide d'un test ELISA de R&D. Les résultats sont exprimés en pg/ml.

**TABLEAU 4**

| | | | Gp160 | Tat natif | gp160 + Tat natif | p24 |
|---|---|---|---|---|---|---|
| Souris contrôle: | | | | | | |
| Souris 1 | j72 | MIP1α | 110 | 100 | 150 | 10 |
| | | MIP1b | 100 | 90 | 140 | 7 |
| Souris 2 | j72 | MIP1α | 120 | 95 | 153 | 8 |
| | | MIP1β | 112 | 80 | 114 | 6 |
| Souris 3 | j72 | MIP1α | 124 | 98 | 128 | 9 |
| | | MIP1β | 99 | 112 | 117 | 7 |

**TABLEAU 5**

Souris immunisées par le Tat toxoïde:

| | | | | | | |
|---|---|---|---|---|---|---|
| Souris 4 | j72 | MIP1α | 152 | 122 | 203 | 10 |
| | | MIP1β | 140 | 118 | 196 | 11 |
| Souris 5 | j72 | MIP1α | 145 | 150 | 215 | 8,5 |

(suite)

| | | | | | | |
|---|---|---|---|---|---|---|
| Souris immunisées par le Tat toxoïde: | | | | | | |
| | | MIP1β | 130 | 160 | 230 | 9 |
| Souris 6 | j72 | MIP1α | 147 | 222 | 290 | 7 |
| | | MIP1β | 152 | 218 | 300 | 9 |

**TABLEAU 6**

| | | | | | | |
|---|---|---|---|---|---|---|
| Souris immunisées par le conjugué gp160-Tat toxoïde : | | | | | | |
| Souris 7 | j72 | MIP1α | 810 | 729 | 1600 | 7,5 |
| | | MIP1β | 1100 | 850 | 2000 | 10 |
| Souris 8 | j72 | MIP1α | 1000 | 821 | 1700 | 8 |
| | | MIP1β | 1125 | 876 | 2100 | 9 |
| Souris 9 | j72 | MIP1α | 975 | 768 | 1685 | 8 |
| | | MIP1β | 1000 | 803 | 1862 | 9 |

Les splénocytes des souris immunisées avec le conjugué gp160-Tat toxoïde produisent plus de chimiokines MIP1α et MIP1β que les cellules des souris immunisées par le Tat toxoïde uniquement lorsqu'elles sont activées, *in vitro,* par les immunogènes utilisés lors de l'immunisation.

**3.2 Production d'IFN gamma dans les surnageants de culture des splénocytes**

La présence d'IFN gamma dans les sumageants de culture des splénocytes cultivés en présence de 5 μg/ml de p24, de gp160, de Tat natif et d'un mélange de 5 μg/ml de gp160 et de 5 μg/ml de Tat natif est déterminée après 72 heures de culture à l'aide d'un test ELISA de R&D. Les résultats sont exprimés en pg/ml.

**TABLEAU 7**

| | | Gp160 | Tat natif | gp160 + Tat natif | p24 |
|---|---|---|---|---|---|
| Souris contrôles : | | | | | |
| Souris 1 | j72 | 0 | 0 | 0 | 0 |
| Souris 2 | j72 | 0 | 0 | 0 | 0 |
| Souris 3 | j72 | 0 | 0 | 0 | 0 |

**TABLEAU 8**

| | | | | | |
|---|---|---|---|---|---|
| Souris immunisées avec le Tat toxoïde | | | | | |
| Souris 4 | j72 | ND | ND | ND | ND |
| Souris 5 | j72 | ND | ND | ND | ND |
| Souris 6 | j72 | ND | ND | ND | ND |

**TABLEAU 9**

| | | | | |
|---|---|---|---|---|
| Souris immunisées avec le conjugué gp160-Tat toxoide : | | | | |
| Souris 7 | j72 | 40 | 600 | 6000 |
| Souris 8 | j72 | 50 | 620 | 5950 |
| Souris 9 | j72 | 65 | 700 | 6850 |

Les splénocytes des souris immunisées avec le conjugué gap160-Tat toxoïde produisent une quantité importante d'IFN gamma lorsqu'elles sont activées, in vitro, avec les immunogènes utilisés lors de l'immunisation.

**3.3. Prolifération des splénocytes de souris immunisées (CMI)**

Les splénocytes des souris immunisées et des souris contrôles sont isolées puis cultivées dans des puits à fond rond d'une plaque de micro-culture à raison de 100 000 cellules/puits en présence de p24, de gp160, de Tat natif et d'un mélange de gp160 et de Tat natif. La culture cellulaire est poursuivie à 37 °C en atmosphère humide chargée à 5 % de CO2 pendant 6 jours. 18 heures avant la fin de l'incubation, 0.5 μCi de thymidine tritiée/ puits sont ajoutés. L'intensité de la réponse immunitaire est proportionnelle à l'index de prolifération Ip.

**Ip = cpm (coups par minute) pour l'antigène donné / cpm contrôle**

**TABLEAU 10**

| | | Gp160 | Tat natif | gp160 + Tat natif | p24 |
|---|---|---|---|---|---|
| Souris contrôle : | | | | | |
| Souris 1 | j72 | 1,2 | 1 | 1,1 | 1 |
| Souris 2 | j72 | 1 | 1,2 | 1 | 1,1 |
| Souris 3 | j72 | 1,1 | 1,1 | 1 | 1 |

**TABLEAU 11**

Souris immunisées avec le Tat toxoïde

| | | Gp160 | Tat natif | gp160 + Tat natif | p24 |
|---|---|---|---|---|---|
| Souris 4 | j72 | 1,2 | 10 | 9 | 1 |
| Souris 5 | j72 | 1 | 8 | 10 | 1,2 |
| Souris 6 | j72 | 1,1 | 9 | 8 | 1,1 |

**TABLEAU 12**

Souris immunisées avec le conjugué gp160-Tat toxoïde :

| | | Gp160 | Tat natif | gp160 + Tat natif | p24 |
|---|---|---|---|---|---|
| Souris 7 | j72 | 8 | 10 | 9 | 1 |
| Souris 8 | j72 | 9 | 8 | 10 | 1 |
| Souris 9 | j72 | 11 | 10 | 9 | 1 |

Les splénocytes des souris immunisées avec le conjugué gp160-Tat toxoïde ou le Tat toxoïde, prolifèrent, lorsqu'elles sont activées, in vitro, avec les immunogènes utilisés lors de l'immunisation.

**Exemple 7 : Activité immunogénique du conjugué [Tat peptides (1-15 ;46-60)] SIAB-[Tat toxoïde]**

A. MATERIEL ET METHODES

L'activité immunogénique de la préparation peptides Tat-Tat toxoïde par rapport à celle du Tat toxoïde seul et son absence de toxicité ont été déterminée chez la souris Balb c de 18-20 g selon les protocoles décrits dans l'exemple 6.

***B. Résultats :***

1-Absence de toxicité du superimmunogène in vivo et in vitro

Les souris immunisées aussi bien par la préparation de peptides Tat-Tat toxoïde que par le Tat toxoïde uniquement ne présentent aucun signe clinique et aucune lésion anatomique. Le test d'immunosuppression montre que des doses de 100 ng/ml à 3 μg/ml de peptides-Tat toxoïde ne diminuent pas la prolifération des lymphocytes.

Aucune des 3 souris immunisées avec 100 μg de la préparation ne manifeste de signes de toxicité (température, troubles cutanés, manifestations systémiques ou régionales) pendant les 7 jours suivant l'injection.

2-Réponse humorale

La réponse humorale est mesurée par la présence dans le sérum d'anticorps de type IgG dirigée contre la protéine recombinante Tat native, mesurée par ELISA et exprimée en titre (inverse de la dilution donnant une densité optique supérieure à 0.3)

**TABLEAU 13**

| | Titre | |
|---|---|---|
| | j-2 | J72 |
| souris contrôle : | | |
| souris 1 | $<500^{-1}$ | $<500^{-}$ |
| souris 2 | $<500^{-1}$ | $<500^{-1}$ |
| souris 3 | $<500^{-1}$ | $<500^{-1}$ |

| | Titre | |
|---|---|---|
| | j-2 | J72 |
| souris immunisées avec le Tat toxoïde : | | |
| souris 4 | $<500^{-1}$ | $32\ 000^{-1}$ |
| souris 5 | $<500^{-1}$ | $48\ 000^{-1}$ |
| souris 6 | $<500^{-1}$ | $48\ 000^{-1}$ |
| souris immunisées avec le Tat peptides-Tat toxoïde : | | |
| souris 7 | $<500^{-1}$ | $64\ 000^{-1}$ |
| souris 8 | $<500^{-1}$ | $64000^{-1}$ |
| souris 9 | $<500^{-1}$ | $64\ 000^{-1}$ |

Les souris immunisées avec le conjugué peptides Tat -Tat toxoïde présentent des titres d'anticorps de type IgG anti-Tat plus important que ceux des souris immunisées avec le Tat toxoïde uniquement.

L'activité neutralisante de ces anticorps a été mesurée à l'aide du Cat assay.

Les résultats sont données en % de neutralisation.

**TABLEAU 14**

Souris immunisées avec le Tat toxoïde :

| | % de neutralisation | | | |
|---|---|---|---|---|
| | 1/50 | 1/100 | 1/200 | 1/400 |
| souris 4 j-2 | 0 | 0 | 0 | 0 |
| j72 | 75 | 50 | 25 | 20 |
| souris 5 j-2 | 0 | 0 | 0 | 0 |
| j72 | 75 | 60 | 30 | 20 |
| souris 6 j-2 | 0 | 0 | 0 | 0 |
| j72 | 75 | 60 | 30 | 20 |

TABLEAU 15

Souris immunisées avec le conjugué peptides Tat - Tat toxoïde

| | 1/50 | 1/100 | 1/200 | 1/400 |
|---|---|---|---|---|
| souris 7 j-2 | 0 | 0 | 0 | 0 |
| j72 | 100 | 100 | 50 | 25 |

(suite)

Souris immunisées avec le conjugué peptides Tat - Tat toxoïde

| | 1/50 | 1/100 | 1/200 | 1/400 |
|---|---|---|---|---|
| souris 8 j-2 | 0 | 0 | 0 | 0 |
| j72 | 100 | 100 | 75 | 50 |
| souris 9 j-2 | 0 | 0 | 0 | 0 |
| j72 | 100 | 100 | 60 | 30 |

Les anticorps induits par le conjugué peptides Tat-Tat toxoïde ont un pouvoir neutralisant plus important que celui induit par le Tat toxoïde.

## Exemple 8 : activité immunogénique du conjugué E7-SIAB-VEGF

### A. MATERIELS ET METHODES

L'activité immunogénique du conjugué E7-SIAB-VEGF par rapport à celle du VEGF et de la protéine E7 et son absence de toxicité ont été déterminées chez la souris C57 black 6 âgée de 6 semaines selon les protocoles décrits dans l'exemple 6.

### *B. Résultats :*

1. Absence de toxicité du superimmunogène in vivo et in vitro

Les souris immunisées aussi bien par la préparation de E7-SIAB-VEGF que par la protéine E7 ou par le VEGF ne présentent aucun signe clinique et aucune lésion anatomique. Le test d'immunosuppression montre que des doses de 100 ng/ml à 3 μg/ml du conjugué E7-SIAB-VEGF ne diminuent pas la prolifération des lymphocytes.

Aucune des 3 souris immunisées avec 100 μg de la préparation ne manifeste de signes de toxicité (température, troubles cutanés, manifestations systémiques ou régionales) pendant les 7 jours suivant l'injection.

2. Réponse humorale

La réponse humorale est mesurée par la présence dans le sérum d'anticorps de type IgG dirigée contre la protéine E7 et contre le VEGF et, mesurée par ELISA et exprimée en titre (inverse de la dilution donnant une densité optique supérieure à 0.3)

**TABLEAU 16**

| | Titre | j-2 | J72 |
|---|---|---|---|
| souris contrôle : | | | |
| souris 1 | E7 | $<500^{-1}$ | $<500^{-1}$ |
| | VEGF | $<500^{-1}$ | $<500^{-1}$ |
| souris 2 | E7 | $<500^{-1}$ | $<500^{-1}$ |
| | VEGF | $<500^{-1}$ | $<500^{-1}$ |
| souris 3 | E7 | $<500^{-1}$ | $<500^{-1}$ |
| | VEGF | $<500^{-1}$ | $<500^{-1}$ |

**TABLEAU 17**

souris immunisées avec le VEGF :

| | | | |
|---|---|---|---|
| souris 4 | E7 | $<500^{-1}$ | $<500^{-1}$ |
| | VEGF | $<500^{-1}$ | $<1000^{-1}$ |
| souris 5 | E7 | $<500^{-1}$ | $<500^{-1}$ |
| | VEGF | $<500^{-1}$ | $1500^{-1}$ |
| souris 6 | E7 | $<500^{-1}$ | $<500^{-1}$ |
| | VEGF | $<500^{-1}$ | $500^{-1}$ |

**TABLEAU 18**

souris immunisées avec le E7

| | | | |
|---|---|---|---|
| souris 7 | E7 | $<500^{-1}$ | $48000^{-1}$ |
| | VEGF | $<500^{-1}$ | $<500^{-1}$ |
| souris 8 | E7 | $<500^{-1}$ | $64000^{-1}$ |
| | VEGF | $<500^{-1}$ | $<500^{-1}$ |
| souris 9 | E7 | $<500^{-1}$ | $48000^{-1}$ |
| | VEGF | $<500^{-1}$ | $<500^{-1}$ |

**TABLEAU 19**

souris immunisées avec le conjugué E7-SIAB-VEGF

| | | | |
|---|---|---|---|
| souris 10 | E7 | $<500^{-1}$ | $48000^{-1}$ |
| | VEGF | $<500^{-1}$ | $48000^{-1}$ |
| souris 11 | E7 | $<500^{-1}$ | $64000^{-1}$ |
| | VEGF | $<500^{-1}$ | $32000^{-1}$ |
| souris 12 | E7 | $<500^{-1}$ | $48000^{-1}$ |
| | VEGF | $<500^{-1}$ | $48000^{-1}$ |

Les souris immunisées avec le conjugué E7-SIAB-VEGF présentent des titres d'anticorps de type IgG anti-VEGF plus importants que ceux des souris immunisées avec le VEGF uniquement, alors que leur réponse anti-E7 reste identique à celle obtenue chez les souris immunisées avec le E7 uniquement.

3.Réponse cellulaire :

3.1 Prolifération des phénocytes de souris immunisées (CMI)

Les splénocytes des souris immunisées et des souris contrôles sont isolées puis cultivées dans des puits à fond rond d'une plaque de micro-culture à raison de 100 000 cellules/puits en présence de E7 natif. La culture cellulaire est poursuivie à 37 °C en atmosphère humide chargée à 5 % de CO2 pendant 6 jours. 18 heures avant la fin de l'incubation, 0.5 µCi de thymidine tritiée par puits sont ajoutés. L'intensité de la réponse immunitaire est proportionnelle à l'index de prolifération Ip

**Ip = cpm (coups par minute) pour l'antigène donné / cpm contrôle**

**TABLEAU 20**

| | | |
|---|---|---|
| Souris contrôle : | | |
| | Souris 1 j72 | 1,2 |
| | Souris 2 j72 | 1 |
| | Souris 3 j72 | 1,1 |
| Souris immunisées avec le VEGF : | | |
| | Souris 4 j72 | 1,2 |
| | Souris 5 j72 | 1 |
| | Souris 6 j72 | 1,1 |
| Souris immunisées avec le E7 : | | |
| | Souris 7 j72 | 6 |
| | Souris 8 j72 | 10 |

(suite)

| | | |
|---|---|---|
| Souris immunisées avec le E7 : | | |
| | Souris 9 j72 | 8 |
| Souris immunisées avec le conjugué E7-SIAB-VEGF | | |
| | Souris 10 j72 | 7 |
| | Souris 11 j72 | 10 |
| | Souris 12 j72 | 9 |

Les splénocytes des souris immunisées avec le conjugué E7-SIAB-VEGF, prolifèrent, lorsqu'elles sont activées, in vitro, avec la protéine E7.

**Exemple 9 : Immunogénicité du conjugué Betv1a-SIAB-IL4(murin)**

**A. MATERIELS ET METHODES**

L'activité immunogénique du conjugué Betv1a-SIAB-Il4 (murin) par rapport à celle du Betv1a et de l'IL4 (murin) et son absence de toxicité ont été déterminées chez la souris Balb c de 18-20 g selon les protocoles décrits dans l'exemple 6.

***B. Résultats :***

1-Absence de toxicité du superimmunogène in vivo et in vitro

Les souris immunisées aussi bien par le conjugué Betv1a-SIAB-IL4 que par l'IL4 uniquement ne présentent aucun signe clinique et aucune lésion anatomique. Le test d'immunosuppression montre que des doses de 100 ng/ml à 3 μg/ml du conjugué Betv 1 a -SIAB-IL4 ne diminuent pas la prolifération des lymphocytes.

Aucune des 3 souris immunisées avec 100 μg de la préparation ne manifeste de signes de toxicité (température, troubles cutanés, manifestations systémiques ou régionales) pendant les 7 jours suivant l'injection.

2.Réponse humorale

La réponse humorale est déterminée par la présence dans le sérum d'anticorps de type IgG dirigée contre le Betv1a et l'IL4, mesurée par ELISA et exprimée en titre (inverse de la dilution donnant une densité optique supérieure à 0.3)

**TABLEAU 21**

| | Titre | j-2 | J72 |
|---|---|---|---|
| souris contrôle : | | | |
| souris 1 | Betv1 a | $<500^{-1}$ | $<500^{-1}$ |
| | IL4 | $<500^{-1}$ | $<500^{-1}$ |
| souris 2 | Betv1a | $<500^{-1}$ | $<500^{-1}$ |
| | IL4 | $<500^{-1}$ | $<500^{-1}$ |
| souris 3 | Betv1a | $<500^{-1}$ | $500^{-1}$ |
| | IL4 | $<500^{-1}$ | $<500^{-1}$ |

**TABLEAU 22**

souris immunisées avec le Betv1a

| | Titre | j-2 | J72 |
|---|---|---|---|
| souris 4 | Betv1a | $<500^{-1}$ | $48000^{-1}$ |
| | IL4 | $<500^{-1}$ | $<500^{-1}$ |
| souris 5 | Betv1a | $<500^{-1}$ | $64000^{-1}$ |
| | IL4 | $<500^{-1}$ | $<500^{-1}$ |
| souris 6 | Betv1a | $<500^{-1}$ | $64000^{-1}$ |

(suite)

| souris immunisées avec le Betv1a | | | |
|---|---|---|---|
| | Titre | j-2 | J72 |
| | IL4 | $<500^{-1}$ | $<500^{-1}$ |
| souris immunisées avec l'IL4 | | | |
| | Titre | j-2 | J72 |
| souris 7 | Betv1a | $<500^{-1}$ | $<500^{-1}$ |
| | IL4 | $< 500^{-1}$ | $500^{-1}$ |
| souris 8 | Betv1a | $<500^{-1}$ | $<500^{-1}$ |
| | IL4 | $<500^{-1}$ | $1500^{-1}$ |
| souris 9 | Betv1a | $<500^{-1}$ | $<500^{-1}$ |
| | IL4 | $<500^{-1}$ | $1000^{-1}$ |

**TABLEAU 23**

| souris immunisées avec le conjugué Betv1a-SIAB-IL4 | | | |
|---|---|---|---|
| | Titre | j-2 | J72 |
| souris 10 | Betv1a | $<500^{-1}$ | $48000^{-1}$ |
| | IL4 | $<500^{-1}$ | $48000^{-1}$ |
| souris 11 | Betv1a | $<500^{-1}$ | $64000^{-1}$ |
| | IL4 | $<500^{-1}$ | $32000^{-1}$ |
| souris 12 | Betv1a | $<500^{-1}$ | $48000^{-1}$ |
| | IL4 | $<500^{-1}$ | $48000^{-1}$ |

Les souris immunisées avec le conjugué Betv1a-SIAB-Il4 présentent des titres d'anticorps de type IgG anti-IL4 plus importants que ceux des souris immunisées avec l'IL4 uniquement, alors que leur réponse anti-Betv1a reste identique à celle obtenue chez les souris immunisées avec le Betv1a uniquement.

**REFERENCES**

Adler HL, McCurdy MA, Kattan MW, Timme TL, Scardino PT, Thompson TC. Elevated levels of circulating interleukin-6 and transforming growth factor-beta1 in patients with metastatic prostatic carcinoma. J Urol 1999;161:182-7

Aucouturier J et al, Adjuvants designed for veterinary and human vaccines. Vaccine 2001 19 :2666-72

Baras B. et al, Single-dose mucosal immunization with biodegradable microparticles containing a Schistosoma mansoni antigen. Infect Immun. (1999) 67:2643-8)

Basak A, Boudreault A, Chen A, Chretien M, Seidah NG, Lazure C., Application of the multiple antigenic peptides (MAP) strategy to the production of prohormone convertases antibodies: synthesis, characterization and use of 8-branched immunogenic peptides. : J Pept Sci 1995 Nov-Dec;1(6):385-95 Chen et al., 1987, Mol. Cell. Biol., 7: 2745-2752.

Cowan et al, Induction of TNF alpha in human neuronal cells by extracellular human T-cell lymphotropic virus Type 1 Tax1, Journal of virology, 1997, 6982-6989.

Ferreira FK et al, 1993, Purification and characterization of recombinant Bet v1, the major Birch pollen allergen : immunological equivalence to natural Bet v1; J. Biol. Chem., 268 : 19574. Flotte et al., 1992, Am. J. Respir, Cell Mol. Biol., 7:349-356.

Fraley et al., J.Bioi.Chem. 255 (1980) 10431

Gopal, 1985, Mol. Cell. Biol., 5 : 1188-1190.

Graham et al., 1973, Virology, 52:456-457.

Harland et al., 1985, J. Cell. Biol., 10 :1094-1095.

Huygen et al., 1996, Nature Medicine, 2(8):893-898

Karayiannakis AJ et al, Serum levels of tumor necrosis factor-alpha and nutritional status in pancreatic cancer patients. Anticancer Res 2001,21:1355-8

Le Buanec et al, HPV-16 E7 but not E6 oncogenic protein triggers both cellular immunosuppression and angiogenic processes. Biomed Pharmacother. 1999;53: 424-31. McLaughlin BA et al., 1996, Am. J. Hum. Genet., 59 : 561-569.

Morgenstem JP et al, Amino acid sequence of Feld1, the major allergen of the domestic cat : protein sequence analysis and cDNA cloning., PNAS, 1991, 88 : 9690

Mori N et al, Interleukine-10 gene expression in adult t-cell leukaemia, Blood, 1996,1035-45.
Nicolau C. et al., 1987, Methods Enzymol., 149:157-76.

Potter et al., 1984, Proc Natl Acad Sci U S A.;81(22):7161-5

Samulski et al., 1989, J. Virol., 63 : 3822-3828.

Sementchenko VI, Schweinfest CW, Papas TS, Watson DK., ETS2 function is required to maintain the transformed state of human prostate cancer cells. Oncogene 1998;17:2883-8

Skaugrud O et al, Biomedical and pharmaceutical applications of alginate and chitosan. Biotechnol Genet Eng Rev 1999;16:23-40
Tacson et al., 1996, Nature Medicine, 2(8):888-892.

Tovey ER et al, Mite faeces are a major source of house dust allergens. Nature, 1981.289: 592-593.
Tur-Kaspa et al, 1986, Mol. Cell. Biol., 6 : 716-718.

Yoshiji H et al, Expression of vascular endothelial growth factor, its receptor, and other angiogenic factors in human breast cancer. Cancer Res 1996, 56 :2013-6

Zagury D et al, Interferon alpha and Tat involvement in the immunosuppression of uninfected T cells and C-C chemokine decline in AIDS. Proc Natl Acad Sci U S A 1998;95 : 3851-6

Zusman I, Sandler B, Gurevich P, Zusman R, Smirnoff P, Tendler Y, Bass D, Shani A, Idelevich E, Pfefferman R, Davidovich B, Huszar M, Glick J. Comparative study of the role of serum levels of p53 antigen and its tumor cell concentration in colon cancer detection. Hum Antibodies Hybridomas. (1996)

## Revendications

**1.** Utilisation d'un superimmunogène composite comprenant deux polypeptides immunogènes distincts couplés entre eux par voie chimique et séparés l'un de l'autre, au sein dudit superimmunogène composite, par une chaîne espaceur, ledit superimmunogène composite comprenant :

- un premier polypeptide (a) immunogène consistant en la protéine gp160 du virus HIV1, détoxiquée ou stabilisée si nécessaire, un fragment immunogène de gp160 ou une protéine immunogène qui en est dérivée ; et
- un second polypeptide (b) immunogène consistant la protéine Tat du virus HIV1, détoxiquée ou stabilisée si nécessaire, un fragment immunogène de Tat ou une protéine immunogène qui en est dérivée,

pour l'obtention d'un médicament à action anti-virale pour la prévention ou le traitement du SIDA, induisant une immunité mucosale ou systémique simultanément à l'encontre de la structure antigénique pathogène et de la protéine circulante locale du stroma.

2. Utilisation selon la revendication 1, **caractérisée en ce que** le superimmunogène composite comprend un premier polypeptide immunogène (a) consistant en la protéine gp160 du HIV1 et un second polypeptide immunogène (b) consistant en la protéine Tat du HIV1 détoxiquée.

3. Utilisation selon l'une des revendications 1 et 2, **caractérisée en ce que** la chaîne espaceur consiste en un peptide espaceur linéaire.

4. Utilisation selon l'une des revendications 1 et 2, **caractérisée en ce que** la chaîne peptidique consiste en un peptide espaceur ramifié.

5. Utilisation selon l'une des revendications 1 et 2, **caractérisée en ce que** la chaîne espaceur est une chaîne de type SIAB ou SMCC.

6. Composé superimmunogène composite comprenant deux polypeptides immunogènes distincts couplés entre eux par voie chimique et séparés l'un de l'autre, au sein dudit superimmunogène composite, par une chaîne espaceur, ledit superimmunogène composite comprenant :

    - un premier polypeptide (a) immunogène consistant en la protéine gp160 du virus HIV1, détoxiquée ou stabilisée si nécessaire, un fragment immunogène de gp160 ou une protéine immunogène qui en est dérivée ; et
    - un second polypeptide (b) immunogène consistant la protéine Tat du virus HIV1, détoxiquée ou stabilisée si nécessaire, un fragment immunogène de Tat ou une protéine immunogène qui en est dérivée,

7. Composé superimmunogène composite selon la revendication 6, **caractérisé en ce que** le superimmunogène composite comprend un premier polypeptide immunogène (a) consistant en la protéine gp160 du HIV1 et un second polypeptide immunogène (b) consistant en la protéine Tat du HIV1 détoxiquée.

8. Composé superimmunogène composite selon l'une des revendications 6 et 7, **caractérisée en ce que** la chaîne espaceur consiste en un peptide espaceur linéaire.

9. Composé superimmunogène composite selon l'une des revendications 6 et 7, **caractérisée en ce que** la chaîne peptidique consiste en un peptide espaceur ramifié.

10. Composé superimmunogène composite selon l'une des revendications 6 et 7, **caractérisée en ce que** la chaîne espaceur est une chaîne de type SIAB ou SMCC.

11. Composition immunogène **caractérisée en ce qu'**elle comprend une quantité immunologiquement efficace d'un superimmunogène composite selon l'une des revendications 6 à 10, en association avec un ou plusieurs excipients physiologiquement compatibles.

12. Vaccin, **caractérisé en ce qu'**il comprend, à titre de principe actif, un superimmunogène composite selon l'une des revendications 6 à 10, en association avec un ou plusieurs excipients physiologiquement compatibles.

**Claims**

1. The use of a composite superimmunogen comprising two distinct immunogenic polypeptides, compled together by chemical treatment and separated one from the other, within the said composite superimmunogen, by a spacer chain, the said composite superimmunogen comprising:

    - a first immunogenic polypeptide (a) consisting of the gp160 protein from the HIV1 virus, which is detoxicated or stabilised if required, an immunogenic fragment of gp160 or an immunogenic protein derived thereof; and
    - a second immunogenic polypeptide (b) consisting of the Tat protein from the HIV1 virus, which is detoxicated or stabilised if required, an immunogenic fragment of Tat or an immunogenic protein derived thereof,

    for obtaining a drug with an anti-viral action for the prevention or for the treatment of AIDS inducing a mucosal or systemic immunity both towards the pathogenic antigenic structure and the local circulating protein of the stroma.

2. The use according to claim 1, **characterized in that** the composite superimmuniogen comprises a first immunogenic

polypeptides (a) consisting of the gp160 protein from HIV1 and a second immunogenic polypeptide (b) consisting of the detoxicated Tat protein from HIV1.

3. The use according to anyone of claim 1 and 2, **characterized in that** the spacer chain consists of a linear spacer peptide.

4. The use according to anyone of claim 1 and 2, **characterized in that** the peptide chain consists of a branched spacer peptide.

5. The use according to anyone of claim 1 and 2, **characterized in that** the spacer chain is a chain of the SIAB or SMCC type.

6. A composite superimmunogen comprising comprising two distinct immunogenic polypeptides, compled together by chemical treatment and separated one from the other, within the said composite superimmunogen, by a spacer chain, the said composite superimmunogen comprising:

- a first immunogenic polypeptide (a) consisting of the gp160 protein from the HIV1 virus, which is detoxicated or stabilised if required, an immunogenic fragment of gP160 or an immunogenic protein derived thereof; and
- a second immunogenic polypeptide (b) consisting of the Tat protein from the HIV1 virus, which is detoxicated or stabilised if required, an immunogenic fragment of Tat or an immunogenic protein derived thereof.

7. The composite superimmunogen according to claim 6, **characterised in that** the said superimmunogen composite comprises a first immunogenic polypeptide (a) consisting of the gp160 protein from HIV1 and a second immunogenic polypeptide consisting of the detoxicated Tat protein from HIV1.

8. The composite superimmunogen according to anyone of claims 6 and 7, **characterised in that** the spacer chain consists of a linear spacer peptide.

9. The composite superimmunogen according to anyone of claims 6 and 7, **characterised in that** the peptide chain consists of a branched spacer peptide.

10. The composite superimmunogen according to anyone of claims 6 and 7, **characterised in that** the said spacer chain is a chain of the SIAB or SMCC type.

11. An immunogenic composition **characterized in that** it comprises an immunologically efficient amount of a composite superimmunogen according to any of claims 6 to 10, in association with one or more physiologically compatible excipients.

12. A vaccine, **characterized in that** it comprises, as an active ingredient, a composite superimmunogen according to any of claims 6 to 10, in association with one or more physiologically compatible excipients.

## Patentansprüche

1. Verwendung eines zusammengesetzten Superimmunogens, das zwei unterschiedliche immunogene Polypeptide umfasst, die chemisch aneinander gekoppelt und innerhalb des zusammengesetzten Superimmunogens durch eine Spacerkette voneinander getrennt sind, wobei das zusammengesetzte Superimmunogen Folgendes umfasst:

- ein erstes immunogenes Polypeptid (a), das aus dem gegebenenfalls detoxifizierten oder stabilisierten Protein gp160 des Virus HIV1, einem immunogenen Fragment von gp160 oder einem davon abgeleiteten immunogenen Protein besteht; und
- ein zweites immunogenes Polypeptid (b), das aus dem gegebenenfalls detoxifizierten oder stabilisierten Tat-Protein des Virus HIV1, einem immunogenen Fragment von Tat oder einem davon abgeleiteten immunogenen Protein besteht;

zur Gewinnung eines Medikaments mit antiviraler Wirkung zur Prävention oder Behandlung von AIDS, das eine mukosale oder systemische Immunität gleichzeitig gegen die pathogene antigene Struktur und das zirkulierende Protein lokal im Stroma induziert.

2. Verwendung gemäß Anspruch 1, **dadurch gekennzeichnet, dass** das zusammengesetzte Superimmunogen ein erstes immunogenes Polypeptid (a), das aus dem Protein gp160 des Virus HIV1 besteht, und ein zweites immunogenes Polypeptid (b), das aus dem detoxifizierten Tat-Protein des HIV1 besteht, umfasst.

3. Verwendung gemäß einem der Ansprüche 1 und 2, **dadurch gekennzeichnet, dass** die Spacerkette aus einem linearen Spacerpeptid besteht.

4. Verwendung gemäß einem der Ansprüche 1 und 2, **dadurch gekennzeichnet, dass** die Peptidkette aus einem verzweigten Spacerpeptid besteht.

5. Verwendung gemäß einem der Ansprüche 1 und 2, **dadurch gekennzeichnet, dass** die Spacerkette eine Kette des Typs SIAB oder SMCC ist.

6. Zusammengesetzte superimmunogene Verbindung, die zwei unterschiedliche immunogene Polypeptide umfasst, die chemisch aneinander gekoppelt und innerhalb des zusammengesetzten Superimmunogens durch eine Spacerkette voneinander getrennt sind, wobei das zusammengesetzte Superimmunogen Folgendes umfasst:

   - ein erstes immunogenes Polypeptid (a), das aus dem gegebenenfalls detoxifizierten oder stabilisierten Protein gp160 des Virus HIV1, einem immunogenen Fragment von gp160 oder einem davon abgeleiteten immunogenen Protein besteht; und
   - ein zweites immunogenes Polypeptid (b), das aus dem gegebenenfalls detoxifizierten oder stabilisierten Tat-Protein des Virus HIV1, einem immunogenen Fragment von Tat oder einem davon abgeleiteten immunogenen Protein besteht.

7. Zusammengesetzte superimmunogene Verbindung gemäß Anspruch 6, **dadurch gekennzeichnet, dass** das zusammengesetzte Superimmunogen ein erstes immunogenes Polypeptid (a), das aus dem Protein gp160 des Virus HIV1 besteht, und ein zweites immunogenes Polypeptid (b), das aus dem detoxifizierten Tat-Protein des HIV1 besteht, umfasst.

8. Zusammengesetzte superimmunogene Verbindung gemäß einem der Ansprüche 6 und 7, **dadurch gekennzeichnet, dass** die Spacerkette aus einem linearen Spacerpeptid besteht.

9. Zusammengesetzte superimmunogene Verbindung gemäß einem der Ansprüche 6 und 7, **dadurch gekennzeichnet, dass** die Peptidkette aus einem verzweigten Spacerpeptid besteht.

10. Zusammengesetzte superimmunogene Verbindung gemäß einem der Ansprüche 6 und 7, **dadurch gekennzeichnet, dass** die Spacerkette eine Kette des Typs SIAB oder SMCC ist.

11. Immunogene Zusammensetzung, **dadurch gekennzeichnet, dass** sie eine immunologisch wirksame Menge eines zusammengesetzten Superimmunogens gemäß einem der Ansprüche 6 bis 10 in Verbindung mit einem oder mehreren physiologisch verträglichen Arzneimittelhilfsstoffen umfasst.

12. Impfstoff, **dadurch gekennzeichnet, dass** er als Wirkstoff ein zusammengesetztes Superimmunogen gemäß einem der Ansprüche 6 bis 10 in Verbindung mit einem oder mehreren physiologisch verträglichen Arzneimittelhilfsstoffen umfasst.

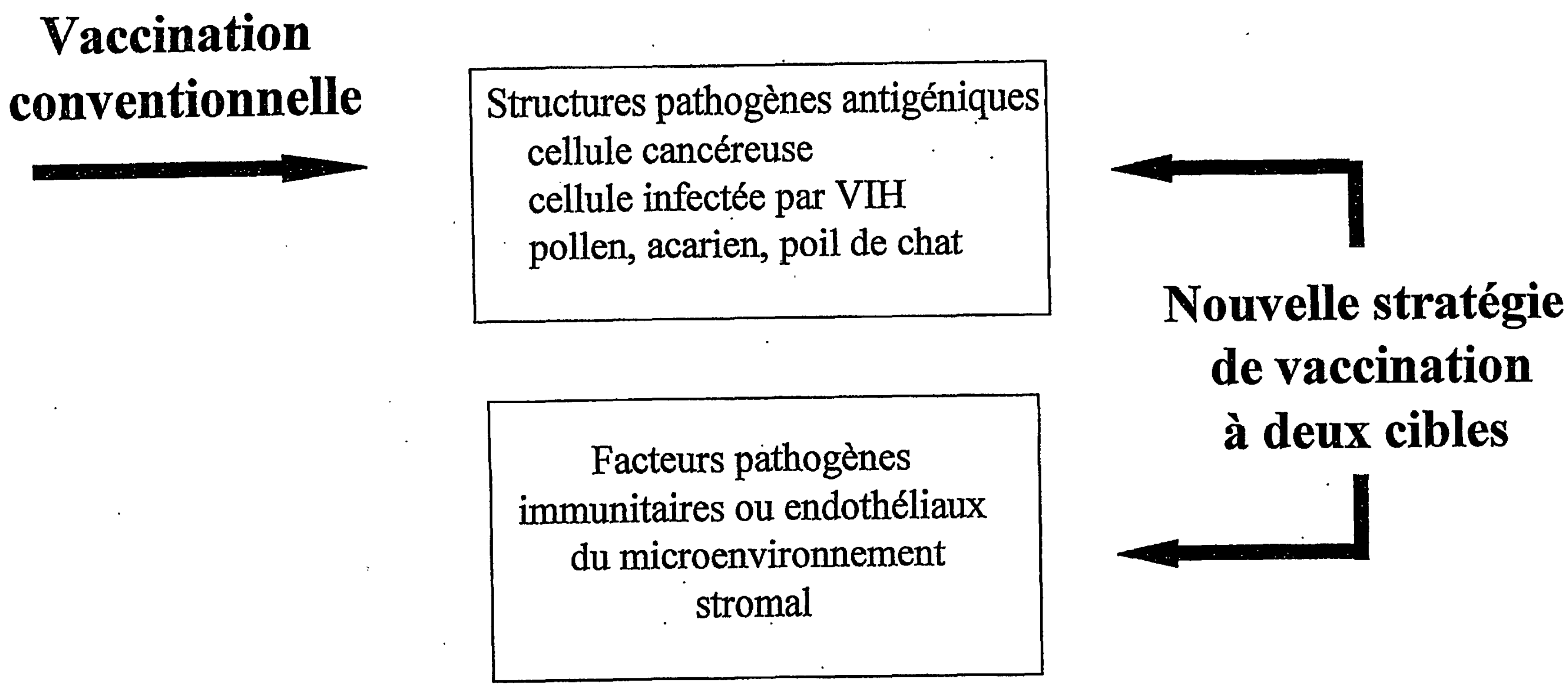
Vaccination conventionnelle
Structures pathogènes antigéniques
cellule cancéreuse
cellule infectée par VIH
pollen, acarien, poil de chat
Nouvelle stratégie de vaccination à deux cibles
Facteurs pathogènes immunitaires ou endothéliaux du microenvironnement stromal

FIGURE 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0003732 A
- WO 9627389 A
- WO 9933872 A
- WO 8606414 A
- EP 0220273 A
- US 8600831 W
- WO 9511307 A


### Littérature non-brevet citée dans la description


- **ZAGURY D et al.** *Proc. Natl. Acad. Sci. USA,* 2001, vol. 98 (14), 8024-8029
- **Ebert EC ; Roberts AI ; O'Connell SM ; Robertson FM ; Nagase H.** Characterization of an immunosuppressive factor dérived from colon cancer cells. *J. Immun ol,* 1987, vol. 138, 2161-8
- **Remacle-Bonn et MM ; Pommier FJ ; Kaplanski S ; Rance RJ ; Depieds RC.** Inhibition of normal allogenic lymphocyte mitogenesis by a soluble inhibitor extracted from human colonic carcinoma. *J. Immunol.,* 1976, vol. 117, 1145-51
- **29-Fontana A ; Hengartner H ; Tribolet N ; Weber E.** Glioblastoma cells release interleukin 1 and factors inhibiting interleukin 2-mediated effects. *J. Immunol.,* 1984, vol. 132, 1837-44
- **30.Hersey P. Bindon ; Czemiecki M ; spurling A ; Wass J ; McCarthy WH.** Inhibition of interleukin 2 production by factors released from tumor cells. *J. Immunol.,* 1983, vol. 131, 2837-42
- **Tamura K ; Shibata Y ; Matsuda Y ; Ishida N.** Isolation and characterization of an immunosuppressive acidid protein from ascitic fluids of cancer patients. *Cancer Res.,* 1981, vol. 41, 3244-52
- *J. Immunol,* 1981, vol. 127, 2300-7
- **Remvikos Y. ; Tominaga O ; Hammel P ; Laurent-Puig P ; Salmon RJ ; Dutrillaux B ; Thomas G.** Increased p53 protein content of colorectal tumours correlates with poor survival. *Br J Cancer,* 1992, vol. 66, 758-64
- **Gan H ; Ouyang Q ; Wang Y.** Expression of p53 protein in colorectal cancer and its relationship to cell proliferative activity and prognosis. *Chung Hua Chung Liu Tsa Chih,* 1996
- **Zusman I ; Sandler B ; Gurevich P ; zusman R ; Smimoff P ; Tendler Y ; Bass D ; Shani A ; Idelevich E ; Pfefferman R.** Comparative study of the role of serum levels of p53 antigen and its tumor cell concentration in colon cancer detection. *Hum Antibodies Hybridomas,* 1996, 123-8
- **Sandler B ; Smimoff P ; Tendelr Y ; Zinder O ; Zusman R ; Zusman I.** Specificity of polyclonal anti-p53 IgG for isolation of the soluble p53 antigen from human serum. *Int J. Mol. Med.,* 1998, vol. 1, 767-70
- **Sadick et al.** *J. Exp. Med.,* 1990, vol. 171, 115-127
- **Frankel et al.** *Cell,* 1988, vol. 55
- **Hermanson G.T.** Bioconjugate techniques. Academic Press, 1996, 239-242
- **Samoszuk M.K. et al.** *Antibody, Immunoconjugates Radiopharm.,* 1989, vol. 2 (1), 37-46
- **McCluskie.** *Microbes and Infection,* 1999, 685-698
- **Boyaka et al.** Strategies for mucosal vaccine development. *Am. J. Trop. Med. Hyg.,* 1999, vol. 60 (4), 35-45
- **Rojas et al.** *Pharmaceutical Research,* 1999, vol. 16 (2), 255
- **Adler HL ; McCurdy MA ; Kattan MW ; Timme TL ; Scardino PT ; Thompson TC.** Elevated levels of circulating interleukin-6 and transforming growth factor-beta1 in patients with metastatic prostatic carcinoma. *J Urol,* 1999, vol. 161, 182-7
- **Aucouturier J et al.** Adjuvants designed for veterinary and human vaccines. *Vaccine,* 2001, vol. 19, 2666-72
- **Baras B. et al.** Single-dose mucosal immunization with biodegradable microparticles containing a Schistosoma mansoni antigen. *Infect Immun.,* 1999, vol. 67, 2643-8
- **Basak A ; Boudreault A ; Chen A ; Chretien M ; Seidah NG ; Lazure C.** Application of the multiple antigenic peptides (MAP) strategy to the production of prohormone convertases antibodies: synthesis, characterization and use of 8-branched immunogenic peptides. *J Pept Sci,* Novembre 1995, vol. 1 (6), 385-95
- **Chen et al.** *Mol. Cell. Biol.,* 1987, vol. 7, 2745-2752

- **Cowan et al.** Induction of TNF alpha in human neuronal cells by extracellular human T-cell lymphotropic virus Type 1 Tax1. *Journal of virology,* 6982-6989
- **Ferreira FK et al.** Purification and characterization of recombinant Bet v1, the major Birch pollen allergen : immunological equivalence to natural Bet v1. *J. Biol. Chem.,* 1993, vol. 268, 19574
- **Flotte et al.** *Am. J. Respir, Cell Mol. Biol.,* 1992, vol. 7, 349-356
- **Fraley et al.** *J.Bioi.Chem.,* 1980, vol. 255, 10431
- **Gopal.** *Mol. Cell. Biol.,* 1985, vol. 5, 1188-1190
- **Graham et al.** *Virology,* 1973, vol. 52, 456-457
- **Harland et al.** *J. Cell. Biol.,* 1985, vol. 10, 1094-1095
- **Huygen et al.** *Nature Medicine,* 1996, vol. 2 (8), 893-898
- **Karayiannakis AJ et al.** Serum levels of tumor necrosis factor-alpha and nutritional status in pancreatic cancer patients. *Anticancer Res,* 2001, vol. 21, 1355-8
- **Le Buanec et al.** HPV-16 E7 but not E6 oncogenic protein triggers both cellular immunosuppression and angiogenic processes. *Biomed Pharmacother,* 1999, vol. 53, 424-31
- **McLaughlin BA et al.** *Am. J. Hum. Genet.,* 1996, vol. 59, 561-569
- **Morgenstem JP et al.** Amino acid sequence of Feld1, the major allergen of the domestic cat : protein sequence analysis and cDNA cloning. *PNAS,* 1991, vol. 88, 9690
- **Mori N et al.** Interleukine-10 gene expression in adult t-cell leukaemia. *Blood,* 1996, 1035-45
- **Nicolau C. et al.** *Methods Enzymol.,* 1987, vol. 149, 157-76
- **Potter et al.** *Proc Natl Acad Sci U S A.,* 1984, vol. 81 (22), 7161-5
- **Samulski et al.** *J. Virol.,* 1989, vol. 63, 3822-3828
- **Sementchenko VI ; Schweinfest CW ; Papas TS ; Watson DK.** ETS2 function is required to maintain the transformed state of human prostate cancer cells. *Oncogene,* 1998, vol. 17, 2883-8
- **Skaugrud O et al.** Biomedical and pharmaceutical applications of alginate and chitosan. *Biotechnol Genet Eng Rev,* 1999, vol. 16, 23-40
- **Tacson et al.** *Nature Medicine,* 1996, vol. 2 (8), 888-892
- **Tovey ER et al.** Mite faeces are a major source of house dust allergens. *Nature,* 1981, vol. 289, 592-593
- **Tur-Kaspa et al.** *Mol. Cell. Biol.,* 1986, vol. 6, 716-718
- **Yoshiji H et al.** Expression of vascular endothelial growth factor, its receptor, and other angiogenic factors in human breast cancer. *Cancer Res,* 1996, vol. 56, 2013-6
- **Zagury D et al.** Interferon alpha and Tat involvement in the immunosuppression of uninfected T cells and C-C chemokine decline in AIDS. *Proc Natl Acad Sci U S A,* 1998, vol. 95, 3851-6
- **Zusman I ; Sandler B ; Gurevich P ; Zusman R ; Smirnoff P ; Tendler Y ; Bass D ; Shani A ; Idelevich E ; Pfefferman R.** Comparative study of the role of serum levels of p53 antigen and its tumor cell concentration in colon cancer detection. *Hum Antibodies Hybridomas,* 1996